# EUROPEAN PATENT APPLICATION

(11) **EP 2 759 264 A1**
(43) Date of publication of application: **30.07.2014**
(21) Application number: 12833823.3
(22) Date of filing: 19.09.2012
(51) Int. Cl.: A61B 6/00, G03B 42/04

(54) **RADIOGRAPHIC SYSTEM, RADIOGRAPHIC SYSTEM COMMUNICATION METHOD, AND RADIOGRAPH-DETECTING EQUIPMENT**

(30) Priority: 20.09.2011 JP 2011204870
(71) Applicant: Fujifilm Corporation, Minato-ku Tokyo 106-8620 (JP)
(72) Inventor: KITAGAWA, Yusuke, Ashigarakami-gun Kanagawa 258-8538 (JP); KAMIYA, Takeshi, Ashigarakami-gun Kanagawa 258-8538 (JP); KUWABARA, Takeshi, Ashigarakami-gun Kanagawa 258-8538 (JP); TAJIMA, Takashi, Ashigarakami-gun Kanagawa 258-8538 (JP)
(74) Representative: Klunker . Schmitt-Nilson . Hirsch
(86) International application number: PCT/JP2012/073886
(87) International publication number: WO 2013/042676

(57) **Abstract**

Communication is performed in an optimal operating environment.

A communication section (80, 81) having a relatively high communication speed is used for communicating a detection signal or an emission stop signal between a source control device (11) and an electronic cassette (13). The detection signal is outputted from a detection pixel (65) of the electronic cassette (13). The emission stop signal depends on a comparison result between an integrated value of the detection signal and an emission stop threshold value. On the other hand, a wireless communication section (37) having a lower communication speed than that of the detection signal and the emission stop signal is used for communicating image data and the like between the electronic cassette (13) and a console (14).

## Description

### TECHNICAL FIELD

The present invention relates to a radiation imaging system, a communication method of the radiation imaging system, and a radiographic image detecting device.

### BACKGROUND ART

In a medical field, an X-ray imaging system using X-rays, as a kind of radiation, is known. The X-ray imaging system is constituted of an X-ray generating apparatus for generating the X-rays and an X-ray imaging apparatus, which receives the X-rays and takes an X-ray image. The X-ray generating apparatus includes an X-ray source for emitting the X-rays to an object, a source control device for controlling the operation of the X-ray source, and an emission switch for inputting an emission start command of the X-rays. The X-ray imaging apparatus includes an X-ray image detecting device and a console. The X-ray image detecting device detects the X-ray image upon receiving the X-rays passed through the object. The console controls the operation of the X-ray image detecting device and applies various image processes to the X-ray image.

Recently, in a field of the X-ray imaging system, an X-ray image detecting device that uses a flat panel detector (FPD) as a detection panel, instead of an X-ray film or an imaging plate (IP), becomes widespread. The FPD has a matrix of pixels each for accumulating signal charge in accordance with the amount of X-rays incident thereon. The FPD accumulates the signal charge on a pixel-by-pixel basis. The FPD converts the accumulated signal charge into a voltage signal at its signal processing circuit, and thereby detects the X-ray image representing image information of the object and outputs the X-ray image as digital image data.

The X-ray image detecting device and the console are connected in a communicatable manner through wired or wireless communication I/Fs. The image data of the X-ray image detected by the X-ray image detecting device is transmitted to the console through the communication I/F. The console transmits information including an imaging condition, various setting commands, and the like to the X-ray image detecting device. The console applies the image processes to the received X-ray image. Then, the console displays the X-ray image on a monitor and stores the X-ray image to an image server.

An electronic cassette (portable X-ray image detecting device) that is composed of the FPD contained in a rectangular parallelepiped housing is in practical use. The electronic cassette is used while being loaded detachably into an existing imaging stand sharable with a film cassette and an IP cassette or a specific imaging stand designed for the electronic cassette, in contrast to a non-detachable type. Furthermore, the electronic cassette is used while being put on a bed or held by the object himself/herself, to take an image of a body part that is hard to take with the non-detachable type. The electronic cassette is sometimes brought out from a hospital to a place having no imaging stand, for use in bedside radiography of an elder patient or in urgent radiography of an injured patient, natural disaster victims, or the like.

Also, the X-ray imaging system performs an automatic exposure control (AEC) of the X-ray image in which the X-ray emission from the X-ray source is stopped as soon as an applied X-ray dose has reached a predetermined threshold value. In the AEC, an AEC-specific dose detection sensor (AEC sensor) for detecting a radiation dose during irradiation with the X-rays, such as an ion chamber is used together with the X-ray image detecting device.

Also, there is a proposed technology for containing such an AEC sensor in the X-ray image detecting device, to eliminate the need for providing the AEC sensor independently of the X-ray image detecting device (patent document 1). According to the patent document 1, the X-ray image detecting device has an output terminal for outputting an AEC signal for stopping the X-ray emission. The X-ray image detecting device is communicatably connected to the source control device through the output terminal. The AEC signal includes a timing signal such as an emission stop signal (interception signal) for stopping the X-ray emission and a dose detection signal representing the radiation dose detected by the AEC sensor. In the case of sending the emission stop signal (timing signal) as the AEC signal from the X-ray image detecting device, the X-ray image detecting device integrates the dose detection signal outputted from the AEC sensor, and compares an integrated value with the threshold value to judge whether or not the integrated value has reached the threshold value. Upon judging that the integrated value has reached the threshold value, the emission stop signal is sent from the X-ray image detecting device to the source control device.

On the other hand, in the case of sending the dose detection signal from the X-ray image detecting device as the AEC signal, the X-ray image detecting device sequentially sends the dose detection signal to the source control device. The source control device performs a series of processes related to the AEC, including integration of the dose detection signal sent from the X-ray image detecting device, comparison between the dose detection signal and the threshold value, and judgment whether or not the integrated value has reached the threshold value.

### PRIOR ART DOCUMENTS

### Patent Documents

Patent Document 1: Japanese Patent No. 4006255

### SUMMARY OF THE INVENTION

### Problems to be Solved by the Invention

In performing the AEC, as described above, the X-ray image detecting device communicates the AEC signal with the source control device, in addition to communication of the image data and the like with the console. The communication of the AEC signal requires rapidity, as compared with the communication of the image data and the like. This is because a delay in a process of stopping the X-ray emission reduces the quality of the X-ray image and causes unnecessary radiation exposure of the patient, owing to an excessive radiation dose beyond an appropriate value. For example, in chest radiography, time from the start of X-ray emission to the stop thereof is extremely short on the order of 50 ms. In such short time, the X-ray image detecting device or the source control device has to perform a series of processes related to the AEC based on the dose detection signal outputted from the AEC sensor, and the source control device has to perform a process for actually stopping the X-ray emission from the X-ray source. Therefore, the communication of the AEC signal between the source control device and the X-ray image detecting device requires rapidity.

On the contrary, the communication of the other information such as the image data between the X-ray image detecting device and the console does not require as much rapidity as the communication of the AEC signal. Instead, since the console is often installed in an operators room partitioned from an examination room, it is required to reduce complicated routing of a communication cable between the X-ray image detecting device and the console. This is a matter of concern especially in the case of using the electronic cassette as the X-ray image detecting device. As described above, the electronic cassette is sometimes used while being detached from the imaging stand. The electronic cassette and the console are sometimes carried about to be shared in a plurality of examination rooms having the X-ray source. In the case of using the electronic cassette in a detached state from the imaging stand or carrying about the electronic cassette, the complicated routing of the communication cable adversely affects the handleability and the portability of the electronic cassette and the console. Therefore, it is required to ease the routing.

The patent document 1 describes no measure against the above requests regarding the communication between the source control device and the X-ray image detecting device and the communication between the X-ray image detecting device and the console.

The present invention aims to provide a radiation imaging system, a communication method of the radiation imaging system, and a radiographic image detecting device that can meet the requests regarding the communication between the source control device and the radiographic image detecting device and the communication between the radiographic image detecting device and the console to establish communication in an optimal operating environment.

### Means for Solving the Problems

A radiation imaging system according to the present invention includes a radiation source, a source control device, a radiographic image detecting device, a console, a high speed communication unit, and a low speed wireless communication unit. The radiation source emits radiation to an object. The source control device controls an operation of the radiation source. The radiographic image detecting device detects a radiographic image by receiving the radiation passed through the object. Furthermore, the radiographic image detecting device has an AEC sensor for performing automatic exposure control that detects a radiation dose passed through the object and stops a radiation emission from the radiation source as soon as an integrated value of the radiation dose has reached a predetermined emission stop threshold value. The console receives the radiographic image detected by the radiographic image detecting device. The high speed communication unit has a relatively high communication speed, and communicates an AEC signal related to the automatic exposure control between the source control device and the radiographic image detecting device. The low speed wireless communication unit has a communication speed lower than the communication speed of the high speed communication unit, and wirelessly communicates a signal other than the AEC signal between the radiographic image detecting device and the console.

The high speed communication unit has small average delay time of data communication, for example. The low speed wireless communication unit has delay time larger than the delay time of the high speed communication unit, for example.

The high speed communication unit performs wireless communication of the AEC signal, for example. The high speed communication unit preferably performs communication of the AEC signal by ad-hoc communications. The low speed wireless communication unit preferably performs communication of the signal other than the AEC signal by infrastructure communications. It is preferable that the radiographic image detecting device directly communicates the AEC signal with the source control device by the ad-hoc communications.

The high speed communication unit may perform wired communication of the AEC signal. The high speed communication unit may also perform wired communication of the signal other than the AEC signal. The radiation imaging system may include a judging section for judging whether or not to perform the automatic exposure control in radiography in accordance with an imaging condition inputted through the console, and a communication switching section for making the high speed communication unit, instead of the low speed wireless communication unit, communicate the signal other than the AEC signal, in a case where the judging section judges that the automatic exposure control is not performed.

The high speed communication unit and the low speed wireless communication unit may be made of different hardware resources. The radiographic image detecting device may include a first control section for performing control of a process and communication of the AEC signal, and a second control section for performing control of a process and communication of the signal other than the AEC signal.

The radiation imaging system may include a low speed wired communication unit for performing wired communication of the signal other than the AEC signal at a communication speed lower than the communication speed of the high speed communication unit.

The AEC signal is preferably one of a dose detection signal of the AEC sensor and an emission stop signal that is outputted as soon as an integrated value of the dose detection signal of the AEC sensor has reached a predetermined emission stop threshold value. The radiographic image detecting device preferably has two modes, including a first AEC mode for transmitting the dose detection signal of the AEC sensor and a second AEC mode for transmitting the emission stop signal to the source control device through the high speed communication unit.

The source control device and the radiographic image detecting device preferably have, as the high speed communication unit, a detection signal I/F for communicating the dose detection signal and an emission signal I/F for communicating the emission stop signal.

The radiographic image detecting device may have a main body and a supplemental device. The main body has an image detector for detecting the radiographic image and the AEC sensor. The supplemental device has the detection signal I/F and the emission signal I/F. In this case, communication between the supplemental device and the main body adopts a same communication method as a communication method of the high speed communication unit.

The radiographic image detecting device is preferably an electronic cassette having a portable housing. It is preferable that the electronic cassette can be driven by a battery contained in the housing.

The radiation imaging system preferably includes a noncontact power feeding device for supplying electric power to recharge the battery. The battery is rechargeable in a state of being contained in the electronic cassette with the electric power from the noncontact power feeding device. It is preferable that the noncontact power feeding device is embedded in a holder of an imaging stand into which the electronic cassette is detachably loaded.

It is preferable that the radiographic image detecting device includes an image detector that has an imaging surface and detects the radiographic image, and the AEC sensor is disposed in the imaging surface.

According to a communication method of a radiation imaging system according to the present invention, the radiation imaging system includes a radiation source for emitting radiation to an object; a source control device for controlling an operation of the radiation source; a radiographic image detecting device for detecting a radiographic image by receiving the radiation passed through the object, the radiographic image detecting device having an AEC sensor for performing automatic exposure control that detects a radiation dose passed through the object and stops a radiation emission from the radiation source as soon as an integrated value of the radiation dose has reached a predetermined emission stop threshold value; and a console for receiving the radiographic image detected by the radiographic image detecting device. The communication method includes a high speed communication step and a low speed wireless communication step. In the high speed communication step, an AEC signal related to the automatic exposure control is communicated at relatively high speed between the source control device and the radiographic image detecting device. In the low speed wireless communication step, a signal other than the AEC signal is wirelessly communicated between the radiographic image detecting device and the console at a communication speed lower than the communication speed of the AEC signal.

A radiographic image detecting device according to the present invention is to be used in combination with a radiation source for emitting radiation to an object and a source control device for controlling an operation of the radiation source, to detect a radiographic image by receiving the radiation passed through the object. The radiographic image detecting device includes an AEC sensor, a high speed communication unit, and a low speed wireless communication unit. The AEC sensor performs automatic exposure control that detects a radiation dose passed through the object and stops a radiation emission from the radiation source as soon as an integrated value of the radiation dose has reached a predetermined emission stop threshold value. The high speed communication unit communicates an AEC signal related to the automatic exposure control with the source control device at a relatively high communication speed. The low speed wireless communication unit wirelessly communicates a signal other than the AEC signal with a console for receiving the radiographic image at a communication speed lower than the communication speed of the AEC signal.

### Effect of the Invention

According to the present invention, the AEC signal is communicated at the relatively high communication speed, and the signal other than the AEC signal is communicated wirelessly at the communication speed lower than the communication speed of the AEC signal. Therefore, it is possible to provide the communication method of the radiation imaging system and the radiographic image detecting device that can make communication in an optimal operating environment.

### BRIEF DESCRIPTION OF DRAWINGS

Fig. 1 is a schematic view showing the structure of an X-ray imaging system;
Fig. 2 is a diagram showing the internal structure of a source control device and the connection relation between the source control device and other devices;
Fig. 3 is a block diagram showing the internal structure of an electronic cassette;
Fig. 4 is a diagram for explaining the disposition of detection pixels in an FPD of the electronic cassette;
Fig. 5 is a block diagram showing the internal structure of an AEC unit and a communication unit of the electronic cassette;
Fig. 6 is a diagram showing imaging conditions set in a console;
Fig. 7 is a block diagram showing the internal structure of the console;
Fig. 8 is a block diagram showing the functions of the console and the flow of information;
Fig. 9 is a table of radiation source information;
Fig. 10 is a comparison table between an easy installation priority type (first AEC mode) and an easy installation non-priority type (second AEC mode);
Fig. 11 is a flowchart of an initial setting process;
Fig. 12 is a flowchart of an AEC execution process in radiography;
Fig. 13 is a diagram showing an operation state of the communication unit and the AEC unit in the first AEC mode in a case where the source control device has no integrator;
Fig. 14 is a diagram showing an operation state of the communication unit and the AEC unit in the first AEC mode in a case where the source control device has an integrator;
Fig. 15 is a diagram showing an operation state of the communication unit and the AEC unit in the second AEC mode;
Fig. 16 is a flowchart of a communication method choosing process;
Fig. 17 is a block diagram in a state where hardware resources of a controller and a communicator related to AEC are operated independently of hardware resources of the other controller and communicator;
Fig. 18 is a diagram showing an example of the structure of a power feeding electrode and a power receiving part of the electronic cassette;
Fig. 19 is a block diagram showing an example of the electronic cassette that is constituted of a cassette main body and a supplemental device;
Fig. 20 is a diagram showing an example of a type selection window to which the type differing from area to area is inputted manually;
Fig. 21 is a diagram for explaining imaging conditions settable in the source control device and a measure against a case where emission stop threshold values of the source control device are less than those of the electronic cassette; and
Fig. 22 is a block diagram showing an example where signals other than an emission stop signal are transmitted and received through a communication I/F, while the emission stop signal is received through an I/F dedicated to the emission stop signal.

### DESCRIPTION OF INVENTION

### First Embodiment

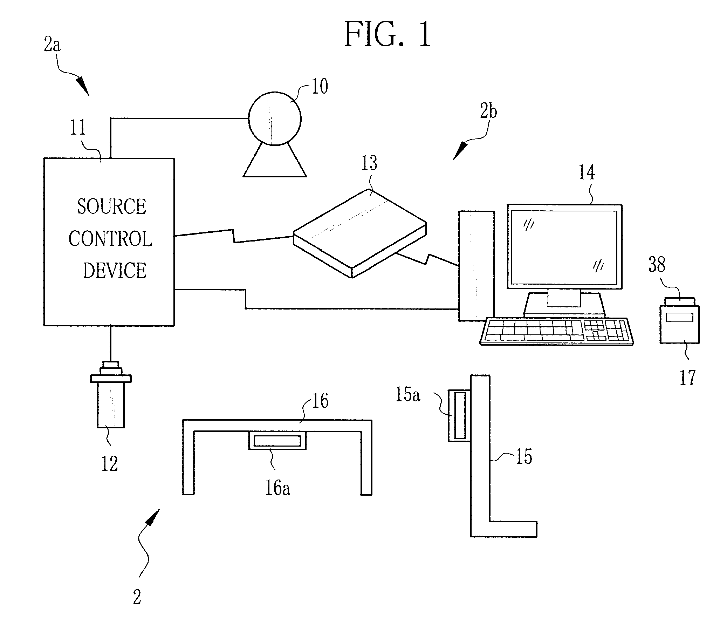

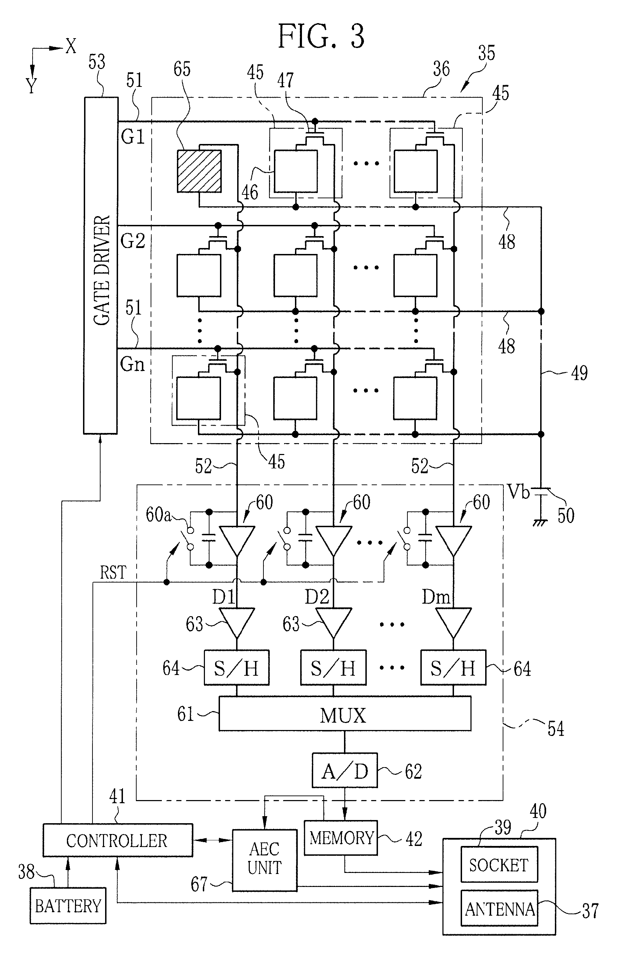

In Fig. 1, an X-ray imaging system (radiation imaging system) 2 includes an X-ray source (radiation source) 10 containing an X-ray tube for radiating X-rays, a source control device 11 for controlling the operation of the X-ray source 10, an emission switch 12 for commanding a start of X-ray emission, an electronic cassette (radiographic image detecting device) 13 for detecting the X-rays passed through an object and outputting an X-ray image, a console 14 for performing operation control of the electronic cassette 13, an image process of the X-ray image, and display of the X-ray image, an imaging stand 15 for imaging the object in a standing position, and an imaging table 16 for imaging the object in a lying position. The X-ray source 10, the source control device 11, and the emission switch 12 compose an X-ray generating apparatus 2a. The electronic cassette 13 and the console 14 compose an X-ray imaging apparatus 2b. In addition to above, the X-ray imaging system 2 is provided with a cradle 17 for recharging a battery 38 (see Fig. 3 too) to be contained in the electronic cassette 13, a source moving device (not shown) for setting the X-ray source 10 in a desired orientation and position, and the like. Note that, the source control device 11 and the console 14 may be integrated into one unit.

The X-ray source 10 has the X-ray tube for radiating the X-rays and an irradiation field limiting device (collimator) for limiting an irradiation field of the X-rays radiating from the X-ray tube. The X-ray tube has a cathode composed of a filament for emitting thermoelectrons, and an anode (target) that radiates the X-rays by collision of the thermoelectrons emitted from the cathode. The irradiation field limiting device is composed of, for example, four lead plates for blocking the X-rays. The four lead plates are disposed in each side of a rectangle so as to form a rectangular irradiation opening in a middle to pass the X-rays therethrough. Shifting the position of the lead plates varies the size of the irradiation opening to limit the irradiation field.

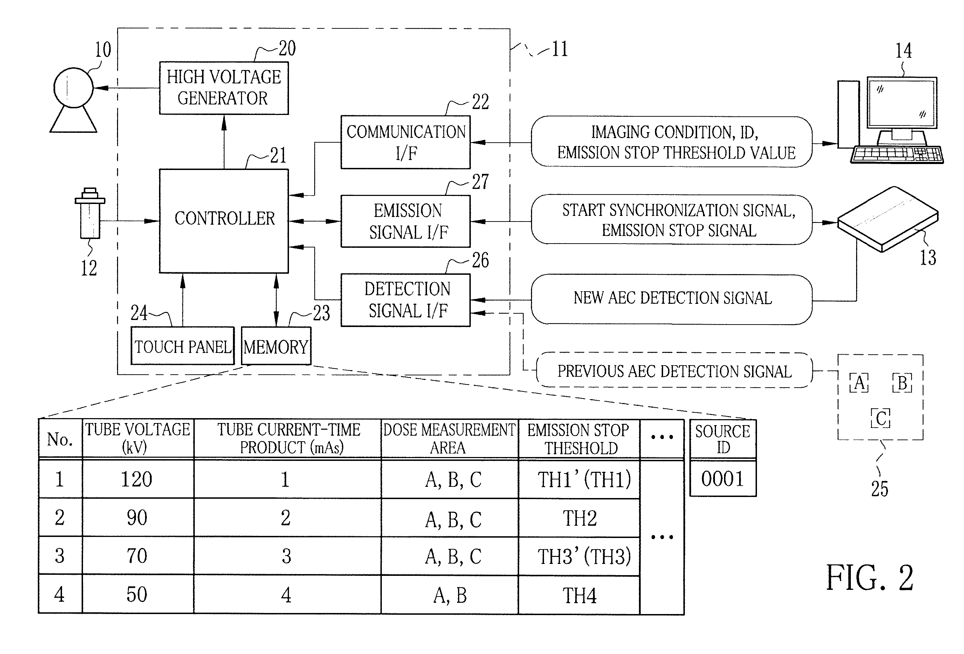

As shown in Fig. 2, the source control device 11 is provided with a high voltage generator 20, a controller 21, and a communication I/F 22. The high voltage generator 20 generates a high tube voltage by multiplying an input voltage using a transformer, and supplies the tube voltage to the X-ray source 10 through a high voltage cable. The controller 21 controls the tube voltage that determines an energy spectrum of the X-rays radiating from the X-ray source 10, a tube current that determines an X-ray emission amount per unit of time, and an X-ray emission time. The communication I/F 22 mediates transmission and reception of principal information and signals to and from the console 14.

To the controller 21, the emission switch 12, a memory 23, and a touch panel 24 are connected. The emission switch 12 is, for example, a two-step press switch to be operated by an operator such as a radiological technician. Upon a first-step press of the emission switch 12, a warm-up start signal is issued to start warming up the X-ray source 10. Upon a second-step press, an emission start signal is issued to make the X-ray source 10 start emitting the X-rays. These signals are inputted to the source control device 11 through a signal cable. Upon receiving the emission start signal from the emission switch 12, the controller 21 starts electric power supply from the high voltage generator 20 to the X-ray source 10.

A radiation dose necessary for obtaining the X-ray image of favorable image quality approximately depends on a body part to be imaged of the object. However, since X-ray transmittance depends on a physique of the object, even if the same radiation dose is applied, a radiation dose received by the electronic cassette 13 varies in accordance with the physique of the object. For this reason, the X-ray imaging system 2 adopts AEC so that the electronic cassette 13 can obtain the necessary radiation dose irrespective of variations in the physique of the object.

To the source control device 11, an AEC sensor 25 is connectable. The AEC sensor 25 is composed of, for example, a well-known ion chamber and the like. The AEC sensor 25 has been used together with a film cassette or an IP cassette to perform the AEC in radiography, since before introducing the X-ray imaging apparatus 2b having the electronic cassette 13. The AEC sensor 25 is a device independent of the electronic cassette 13, and outputs a dose detection signal representing an incident radiation dose as the AEC signal.

As described later on, the electronic cassette 13 has another integral AEC sensor, and the AEC sensor 25 is not used in the case of using the electronic cassette 13. To distinguish between the AEC sensor 25 and the integral AEC sensor embedded in the electronic cassette 13, the AEC sensor 25 is hereafter called a previous AEC sensor. To distinguish between a dose detection signal outputted from the previous AEC sensor 25 and a dose detection signal outputted from the AEC sensor embedded in the electronic cassette 13, the dose detection signal outputted from the previous AEC sensor 25 is called a previous AEC detection signal, while the dose detection signal outputted from the AEC sensor embedded in the electronic cassette 13 is called a new AEC detection signal.

The previous AEC sensor 25 detects the incident radiation dose as a voltage value, and outputs the detected voltage value as the previous AEC detection signal. The previous AEC sensor 25 repeats detecting the radiation dose in a predetermined sampling cycle. The previous AEC detection signal outputted from the previous AEC sensor 25 may be a voltage value (instantaneous value) obtained by one-time detection of the radiation dose, or an integrated value of the voltage value obtained by plural-time detection of the radiation dose. The integrated value represents an accumulative dose of the incident radiation dose. In the case of outputting the integrated value, the previous AEC sensor 25 is provided with an integrator. The previous AEC sensor 25 updates the integrated value whenever detecting the radiation dose, and outputs the updated integrated value as the previous AEC detection signal.

The previous AEC sensor 25 is of approximately the same size in plane as the size the cassette usable in the X-ray imaging system 2, and is used in a state of being disposed in front of an imaging surface of the cassette. The previous AEC sensor 25 has, for example, three dose measurement areas A, B, and C at upper left and upper right corresponding to lungs in chest radiography and at lower middle, respectively. The previous AEC sensor 25 can output the previous AEC detection signal of each dose measurement area, or a sum value or an average value of the previous AEC detection signals of the plurality of dose measurement areas, depending on its setting.

A detection signal I/F 26 is a connection I/F for connecting the previous AEC sensor 25, and receives the previous AEC detection signal (dose detection signal). The detection signal I/F 26 can receive a signal that is in the same format as the format of the previous AEC detection signal. In the case of using the electronic cassette 13, the detection signal I/F 26 can receive the new AEC detection signal (dose detection signal) outputted from the AEC sensor embedded in the electronic cassette 13.

The detection signal I/F 26 inputs the received previous AEC detection signal to the controller 21. Upon receiving the emission start signal from the emission switch 12, the controller 21 starts monitoring the previous AEC detection signal. The controller 21 compares the integrated value of the previous AEC detection signal with an emission stop threshold value set in an imaging condition at appropriate timing. To be more specific, the controller 21 repeats the comparison between the previous AEC detection signal and the emission stop threshold value whenever receiving the previous AEC detection signal from the previous AEC sensor 25.

The controller 21 continues the X-ray emission from the X-ray source 10, until the previous AEC detection signal reaches the emission stop threshold value. As soon as the previous AEC detection signal has reached the emission stop threshold value, the controller 21 sends an emission stop command to the high voltage generator 20 to stop the X-ray emission. The high voltage generator 20 stops supplying the electric power to the X-ray source 10 in response to the emission stop command, and stops the X-ray emission.

The controller 21 performs the same process as the process of the previous AEC detection signal also in a case where the detection signal I/F 26 receives the new AEC detection signal outputted from the AEC sensor embedded in the electronic cassette 13.

The memory 23 stores in advance a plurality of types of imaging conditions, each including a tube voltage and a tube current-time product (mAs value) preset in the source control device 11. In this embodiment, the tube current-time product, the dose measurement areas of the previous AEC sensor 25, the emission stop threshold value to judge the stop of X-ray emission by comparison with the previous AEC detection signal outputted from the previous AEC sensor 25, and the like are stored as the imaging condition corresponding to each number (No.) and tube voltage (each of four types of 120 kV of No. 1, 90 kV of No. 2, 70 kV of No. 3, and 50 kV of No. 4) of the imaging condition. As the emission stop threshold value, default values TH1 to TH4 are set in advance in shipping the X-ray source 10. As shown in No. 1 having a tube voltage of 120 kV and No. 3 having a tube voltage of 70 kV, if the operator adjusts the default value (TH1 and TH3) during use, both an adjusted value (TH1' and TH3') and the default value (TH1 and TH3) are stored. The imaging condition is set manually by the operator by designating the number (No.) of the imaging condition through the touch panel 24. The item of the dose measurement area includes dose measurement area designating information, which represents which of the three dose measurement areas A to C provided in the previous AEC sensor 25 to use.

The source control device 11 starts the X-ray emission with the tube voltage and the tube current-time product corresponding to the designated number (No.) of the imaging condition. As soon as the AEC detects that the incident radiation dose has reached a sufficient target dose, the AEC stops the X-ray emission even if the tube current-time product has not yet reached the value designated in the imaging condition. Note that, in order to prevent a shortage of the incident radiation dose caused by completion of the X-ray emission before the AEC judges the stop of X-ray emission using the target dose, a value having a margin adequate for the target dose is set as the imaging condition of the X-ray source 10. The value having the margin is, for example, a maximum value allowable under safety restrictions. Note that, the tube current-time product is preferably set at a value in accordance with the body part to be imaged. Instead of the tube current-time product, the tube current and the X-ray emission time may be set separately.

The memory 23 also stores an ID (source ID) to identify a model of the X-ray generating apparatus 2a. The source ID is used for establishing a setting of the X-ray imaging apparatus 2b, which is used together with the X-ray generating apparatus 2a, in accordance with the model of the X-ray generating apparatus 2a. In installing the X-ray imaging apparatus 2b, the console 14 and the source control device 11 are connected communicatably. Upon establishing the communication with the console 14, the controller 21 sends the source ID read from the memory 23, together with information of the emission stop threshold value being the imaging condition, to the console 14 through the communication I/F 22.

An emission signal I/F 27 is used when using the electronic cassette 13, for sending and receiving a start synchronization signal for synchronization between a time of starting the X-ray emission from the X-ray source 10 and a time of starting the operation of the electronic cassette 13. The controller 21 sends and receives the start synchronization signal to and from the electronic cassette 13, upon receiving the warm-up start signal from the emission switch 12.

More specifically, the controller 21 sends to the electronic cassette 13 through the emission signal I/F 27 an emission start request signal, which inquires whether or not the electronic cassette 13 is ready for a start of the X-ray emission. Upon receiving the emission start request signal, the electronic cassette 13 completes a reset process described later on, and performs a preparation process including an accumulation start process and the like. Then, upon receiving through the emission signal I/F 27 an emission permission signal being a response of the emission start request signal from the electronic cassette 13 and further receiving the emission start signal from the emission switch 12, the controller 21 starts supplying the electric power from the high voltage generator 20 to the X-ray source 10. Upon stopping the X-ray emission, the controller 21 sends an emission stop signal to the electronic cassette 13 through the emission signal I/F 27.

The electronic cassette 13 has two AEC modes to perform the AEC, i.e. a first AEC mode and a second AEC mode. An output format and an output I/F of the AEC signal from the electronic cassette 13 to the source control device 11 differ from mode to mode. In the first AEC mode, the new AEC detection signal (dose detection signal) similar to the previous AEC detection signal (dose detection signal) outputted from the previous AEC sensor 25 is outputted. In the first AEC mode, the new AEC detection signal outputted from the electronic cassette 13 is sent to the detection signal I/F 26 of the source control device 11, just as with the previous AEC detection signal outputted from the previous AEC sensor 25. The source control device 11 performs the comparison with the emission stop threshold value based on the received new AEC detection signal.

In the second AEC mode, the emission stop signal (timing signal) for regulating emission stop timing is outputted as the AEC signal. The emission stop signal is received not by the detection signal I/F 26 but by the emission signal I/F 27. In the second AEC mode, the electronic cassette 13 compares the integrated value of the new AEC detection signal with the emission stop threshold value and sends the emission stop signal to the source control device 11 when the integrated value has reached the emission stop threshold value, instead of sending the new AEC detection signal outputted from the integral AEC sensor to the source control device 11. In other words, in the second AEC mode, the electronic cassette 13 performs a process that is performed by the source control device 11 in the first AEC mode based on the previous AEC detection signal or the new AEC detection signal.

Upon receiving by the emission signal I/F 27 the emission stop signal from the electronic cassette 13, the controller 21 of the source control device 11 stops supplying the electric power from the high voltage generator 20 to the X-ray source 10 to stop the X-ray emission. As shown in Fig. 2, in a case where the electronic cassette 13 performs the AEC in the first AEC mode, both the emission signal I/F 27 and the detection signal I/F 26 are connected to the electronic cassette 13. The electronic cassette 13 outputs the new AEC detection signal in the first AEC mode, so the emission signal I/F 27 is used only for transmitting and receiving the synchronization signal for synchronization of an emission start timing. The detection signal I/F 26 is used for receiving the new AEC detection signal from the electronic cassette 13.

On the other hand, in the second AEC mode, the electronic cassette 13 outputs the emission stop signal as the AEC signal. The emission stop signal is received by the emission signal I/F 27, which is used for transmitting and receiving the synchronization signal. Accordingly, only the emission signal I/F 27 is used, and the detection signal I/F 26 is unused in the second AEC mode.

In Fig. 3, as is widely known, the electronic cassette 13 is composed of a flat panel detector (FPD) 35 and a portable housing for containing the FPD 35. The housing of the electronic cassette 13 is in an approximately rectangular and flat shape, and of the same size (a size compatible with International Standard ISO4090:2001) as the size of the film cassette and the IP cassette (also called a CR cassette) in plane. Therefore, the electronic cassette 13 is attachable to an existing imaging stand or table designed for the film cassette and the IP cassette.

A plurality of electronic cassettes 13 are provided in each examination room installed with the X-ray imaging system 2, for example, one electronic cassette 13 for the imaging stand 15 and one electronic cassette 13 for the imaging table 16. The electronic cassette 13 is detachably set in a holder 15a, 16a (see Fig. 1) of the imaging stand 15 or the imaging table 16 in such a position that an imaging surface 36 of the FPD 35 is opposed to the X-ray source 10. The electronic cassette 13 can be used separately from the imaging stand 15 or the imaging table 16 in a state of being put on a bed under the object lying or held by the object himself/herself.

The electronic cassette 13 contains an antenna 37 and a battery 38, and can have wireless communication with the console 14. The antenna 37 transmits and receives a radio wave for use in the wireless communication to and from the console 14. As a wireless communication method between the electronic cassette 13 and the console 14, one having a relatively low communication speed and requiring lower power consumption, for example, a wireless LAN, Bluetooth (trademark), Zigbee (trademark), or the like is available. The battery 38 supplies the electric power to operate each part of the electronic cassette 13. The battery 38 is of a relatively small type so as to be contained in the slim electronic cassette 13. As shown in Fig. 1, the battery 38 can be taken out of the electronic cassette 13 and set in the specific cradle 17 for recharging.

The electronic cassette 13 is provided with a socket 39 in addition to the antenna 37. The socket 39 is provided for having wired communication with the console 14, and used in the case of a malfunction of the wireless communication between the electronic cassette 13 and the console 14 owing to poor signal quality. Upon connecting a cable of the console 14 to the socket 39, the wired communication is established between the electronic cassette 13 and the console 14. Note that, the console 14 may feed power to the electronic cassette 13 using a power feedable multi-cable as the communication cable. This allows operating the electronic cassette 13 and recharging the battery 38 by the power fed by the console 14, even in the case of running out of the battery 38.

The antenna 37 and the socket 39 are provided in a communication unit 40. The communication unit 40 mediates transmission and reception of various types of information including image data and signals (including a life check signal for checking whether or not communication is performed normally and the like) between the antenna 37 or the socket 39 and a controller 41, and between the antenna 37 or the socket 39 and a memory 42. The antenna 37 functions as a low speed wireless communicator, and the socket 39 functions as a low speed wired communicator.

The FPD 35 has a TFT active matrix substrate. In the substrate, a plurality of pixels 45 each for accumulating signal charge in accordance with an X-ray amount incident thereon are arranged to form the imaging surface 36. The plurality of pixels 45 are arranged into a two-dimensional matrix with n rows (X direction) and m columns (Y direction) at a predetermined pitch. "n" and "m" are integers of two or more. The pixel number of the FPD 35 is, for example, approximately 2000 by approximately 2000.

The FPD 35 is of an indirect conversion type, having a scintillator (phosphor) for converting the X-rays into visible light. The pixels 45 perform photoelectric conversion of the visible light converted by the scintillator. The scintillator is made of CsI (cesium iodide), GOS (gadolinium oxysulfide), or the like, and is opposed to the entire imaging surface 36 having the matrix of pixels 45. Note that, the scintillator and the FPD 35 may adopt either a PSS (penetration side sampling) method in which the scintillator and the FPD 35 are disposed in this order from an X-ray incident side, or an ISS (irradiation side sampling) method in which the FPD 35 and the scintillator are disposed in this order oppositely to the PSS method. Also, a direct conversion type FPD, which has a conversion layer (amorphous selenium) or the like for directly converting the X-rays into the electric charge, may be used instead of the scintillator.

The pixel 45 is composed of a photodiode 46, a capacitor (not shown), and a thin film transistor (TFT) 47. The photodiode 46, being a photoelectric conversion element, produces the electric charge (electron and hole pairs) upon entry of the visible light. The capacitor accumulates the electric charge produced by the photodiode 46. The thin film transistor 47 functions as a switching element.

The photodiode 46 is composed of a semiconducting layer (of a PIN type, for example) for producing the electric charge and an upper electrode and a lower electrode disposed on top and bottom of the semiconducting layer. The lower electrode of the photodiode 46 is connected to the TFT 47. The upper electrode of the photodiode 46 is connected to a bias line 48. There are the same number of bias lines 48 provided as the number (n rows) of the rows of the pixels 45 in the imaging surface 36. All the bias lines 48 are coupled to a bus 49. The bus 49 is connected to a bias power supply 50. A bias voltage Vb is applied from the bias power supply 50 to the upper electrodes of the photodiodes 46 through the bus 49 and the bias lines 48. Since the application of the bias voltage Vb produces an electric field in the semiconducting layer, the electric charge (electron and hole pairs) produced in the semiconducting layer by the photoelectric conversion is attracted to the upper and lower electrodes, one of which has a positive polarity and the other of which has a negative polarity. Thereby, the electric charge is accumulated in the capacitor.

A gate electrode of the TFT 47 is connected to a scan line 51. A source electrode of the TFT 47 is connected to a signal line 52. A drain electrode of the TFT 47 is connected to the photodiode 46. The scan lines 51 and the signal lines 52 are routed into a lattice. The number of the scan lines 51 coincides with the number of the rows (n rows) of the pixels 45. The number of the signal lines 52 coincides with the number of the columns (m columns) of the pixels 45. The scan lines 51 are connected to a gate driver 53, and the signal lines 52 are connected to a signal processor 54.

The gate driver 53 drives the TFTs 47 to carry out a charge accumulation operation for accumulating the signal charge in the pixel 45 in accordance with the amount of the X-rays incident thereon, a readout operation (actual readout operation) for reading out the signal charge from the pixels 45, and a reset operation (idle readout operation). The controller 41 controls a start timing of each of the above operations carried out by the gate driver 53.

In the charge accumulation operation, the signal charge is accumulated in the pixels 45 while the TFTs 47 are turned off. In the readout operation, the gate driver 53 sequentially issues gate pulses G1 to Gn each of which drives the TFTs 47 of the same row at a time. Thereby, the scan lines 51 are activated one by one to turn on the TFTs 47 connected to the activated scan line 51 on a row-by-row basis. Upon turning on the TFT 47, the electric charge accumulated in the capacitor of the pixel 45 is read out to the signal line 52 and inputted to the signal processor 54.

Dark charge occurs in the semiconducting layer of the photodiode 46 irrespective of the presence or absence of entry of the X-rays. Due to the application of the bias voltage Vb, the dark charge is accumulated in the capacitor. The dark charge occurring in the pixels 45 becomes noise of the image data, and therefore the reset operation is carried out to remove the dark charge. The reset operation is an operation to discharge the dark charge occurring in the pixels 45 through the signal lines 52.

The reset operation adopts a sequential reset method, for example, by which the pixels 45 are reset on a row-by-row basis. In the sequential reset method, as with the readout operation of the signal charge, the gate driver 53 sequentially issues the gate pulses G1 to Gn to the scan lines 51 to turn on the TFTs 47 of the pixels 45 on a row-by-row basis. While the TFT 47 is turned on, the dark charge flows from the pixel 45 through the signal line 52 into an integration amplifier 60. In the reset operation, in contrast to the readout operation, a MUX 61 does not read out the electric charge accumulated in the integration amplifier 60. In synchronization with the issue of each of the gate pulses G1 to Gn, the controller 41 outputs reset pulses RST to reset the integration amplifiers 60.

Instead of the sequential reset method, a parallel reset method or all pixels reset method may be used. In the parallel reset method, a plurality of rows of pixels are grouped together, and sequential reset is carried out in each group, so as to concurrently discharge the dark charge from the rows of the number of the groups. In the all pixels reset method, the gate pulse is inputted to every row to discharge the dark charge from every pixel at a time. The parallel reset method and the all pixels reset method allow speeding up the reset operation.

The signal processor 54 includes the integration amplifiers 60, the multiplexer (MUX) 61, an A/D converter (A/D) 62, and the like. The integration amplifier 60 is connected to each signal line 52 on a one-by-one basis. The integration amplifier 60 is composed of an operational amplifier and a capacitor connected between input and output terminals of the operational amplifier. The signal line 52 is connected to one of the input terminals of the operational amplifier. The other input terminal of the operational amplifier is connected to a ground (GND). The integration amplifier 60 converts by integration the electric charge inputted from the signal line 52 into each of voltage signals D1 to Dm, and outputs each of the voltage signals D1 to Dm. An output terminal of the integration amplifier 60 of each column is connected to the MUX 61 through another amplifier 63 and a sample holder (S/H) 64. An output of the MUX 61 is connected to the A/D 62.

The MUX 61 sequentially selects one of the plurality of integration amplifiers 60 connected in parallel, and inputs the voltage signals D1 to Dm outputted from the selected integration amplifiers 60 in series to the A/D 62.

The A/D 62 converts the inputted analog voltage signals D1 to Dm of one row into digital values, and outputs the digital values to the memory 42 embedded in the electronic cassette 13. The memory 42 stores the digital values of one row with being associated with coordinates of individual pixels 45 as image data of one row of the X-ray image. Thereby, the readout operation of one row is completed.

After the MUX 61 reads out from the integration amplifiers 60 the voltage signals D1 to Dm of one row, the controller 41 outputs the reset pulse RST to the integration amplifiers 60 to turn on reset switches 60a. Thus, the signal charge of one row accumulated in the integration amplifiers 60 is reset. Upon resetting the integration amplifiers 60, the gate driver 53 outputs the gate pulse of the next row to start reading out the signal charge from the pixels 45 of the next row. By sequential repetition of this operation, the signal charge is read out from the pixels 45 of every row.

After completion of the readout from every row, the image data representing the X-ray image of one frame is stored in the memory 42. This image data is read out from the memory 42, and outputted to the console 14 through the communication unit 40. Thereby, the X-ray image of the object is detected.

Upon receiving the emission start request signal from the controller 21 of the source control device 11, the controller 41 of the electronic cassette 13 makes the FPD 35 perform the reset operation and sends the emission permission signal to the source control device 11. Upon receiving the emission start signal, the controller 41 of the electronic cassette 13 shifts the operation of the FPD 35 from the reset operation to the charge accumulation operation.

The FPD 35 has, in the same imaging surface 36, a plurality of detection pixels 65 each of which is connected to the signal line 52 without through the TFT 47, in addition to the pixels 45 each connected to the signal line 52 through the TFT 47. The detection pixels 65 are pixels for use in detecting the X-ray dose applied to the imaging surface 36 through the object. The detection pixels 65 function as an AEC sensor, just as with the previous AEC sensor 25. The detection pixels 65 occupy, for example, on the order of approximately 0.01% of the pixels 45 in the imaging surface 36.

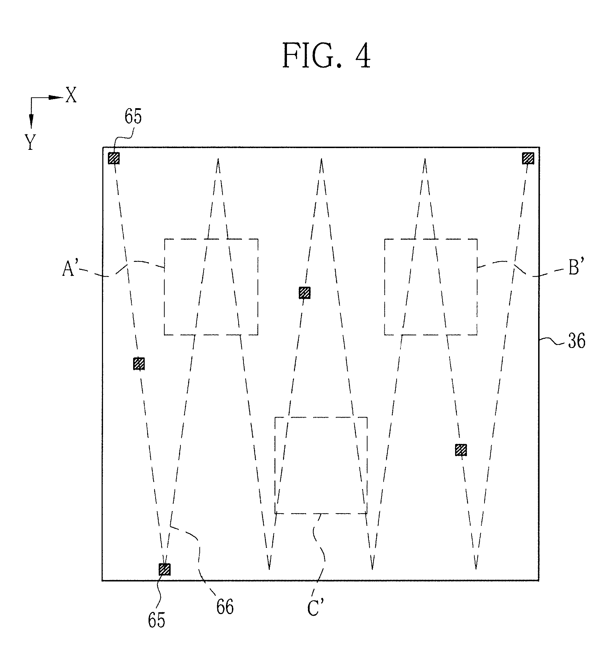

As shown in Fig. 4, the detection pixels 65 are disposed along a waveform line 66 that is horizontally symmetric with respect to the center of the imaging surface 36 as shown by a broken line, so as to be uniformly distributed in the imaging surface 36 without being localized. For example, one detection pixel 65 is laid out in the column of the pixels 45 connected to the single signal line 52. The columns having the detection pixel 65 are arranged at intervals of two to three columns without having the detection pixel 65. The positions of the detection pixels 65 are known in manufacturing the FPD 35, and the FPD 35 has a nonvolatile memory (not shown) that stores the position (coordinates) of every detection pixel 65 in advance.

Since the detection pixel 65 is connected to the signal line 52 directly without through the TFT 47, the signal charge produced in the detection pixel 65 immediately flows into the signal line 52. The same holds true, even while the TFTs 47 of the normal pixels 45 of the same column are turned off and the normal pixels 45 of the same column are in the charge accumulation operation. Thus, the electric charge produced in the detection pixel 65 always flows into the integration amplifier 60 in the signal line 52 connected to the detection pixel 65. During the charge accumulation operation, the electric charge that is produced by the detection pixel 65 and accumulated in the integration amplifier 60 is outputted as a voltage value (new AEC detection signal) through the MUX 61 to the A/D 62 at predetermined sampling intervals. The new AEC detection signal is outputted from the A/D 62 to the memory 42. The memory 42 stores the new AEC detection signal in correspondence with the coordinate information of each detection pixel 65 in the imaging surface 36. The FPD 35 repeats this dose detection operation in the execution of the AEC.

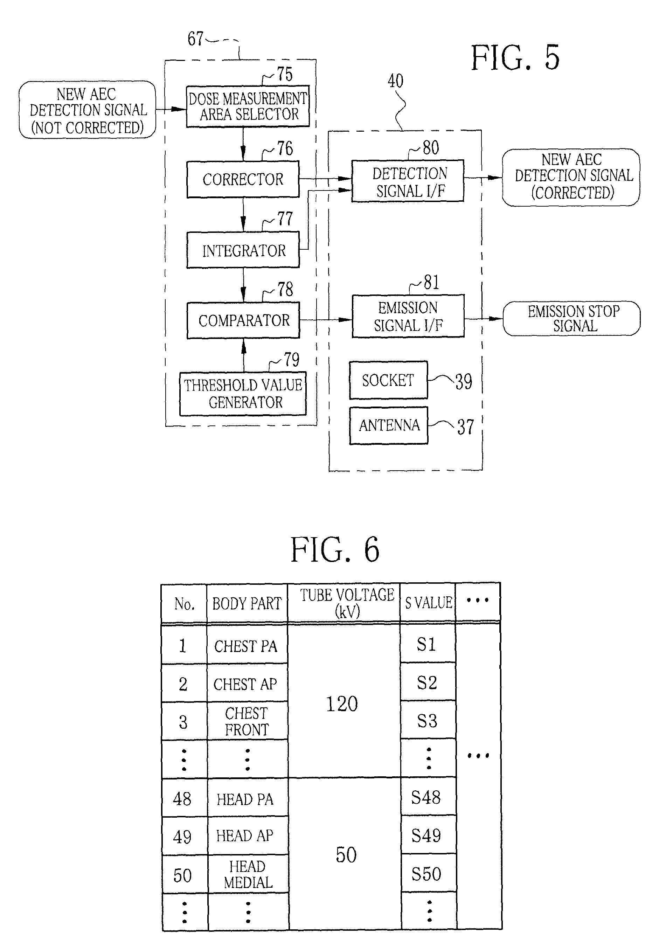

The controller 41 controls the operation of an AEC unit 67. The AEC unit 67 accesses the memory 42 to read out the recorded new AEC detection signal. In Fig. 5, the AEC unit 67 has a dose measurement area selector 75, a corrector 76, an integrator 77, a comparator 78, and a threshold value generator 79.

The dose measurement area selector 75 selects which signal to use in the AEC out of the new AEC detection signals of the plurality of detection pixels 65 distributed in the imaging surface 36, based on the coordinate information of the new AEC detection signals read out of the memory 42. The corrector 76 corrects the new AEC detection signal to a value corresponding to the previous AEC detection signal.

As described later on, the previous AEC sensor 25 and the detection pixel 65 embedded in the electronic cassette 13 are different from each other in sensitivity, a range of an outputted voltage value, and the like. Thus, even if the same X-ray dose is applied, an output value of the previous AEC sensor 25 is different from an output value of the detection pixel 65. In the electronic cassette 13, in the case of executing the AEC in the first AEC mode, the new AEC detection signal is sent to the detection signal I/F 26 of the source control device 11, just as with the previous AEC detection signal. The source control device 11 has no function of distinguishing which one of the previous AEC sensor 25 and the electronic cassette 13 has outputted the detection signal. Accordingly, the corrector 76 corrects the output value of the new AEC detection signal so as to equalize the output value of the new AEC detection to the output value of the previous AEC detection signal.

In the first AEC mode, in the case of outputting the new AEC detection signal as an instantaneous value to the source control device 11, the new AEC detection signal outputted from the corrector 76 is sent to the source control device 11. In this case, neither the integrator 77, the comparator 78, nor the threshold value generator 79 works.

The integrator 77 integrates the new AEC detection signal. In the first AEC mode, in the case of outputting the new AEC detection signal as an integrated value to the source control device 11, the new AEC detection signal outputted from the integrator 77 is sent to the source control device 11. In this case, neither the comparator 78 nor the threshold value generator 79 works.

The comparator 78 and the threshold value generator 79 work in the second AEC mode. In the second AEC mode, upon detecting the start of X-ray emission, the comparator 78 starts monitoring the integrated value of the detection signal from the integrator 77. The comparator 78 compares the integrated value with the emission stop threshold value provided by the threshold value generator 79 in appropriate timing. At the moment when the integrated value has reached the threshold value, the comparator 78 issues the emission stop signal.

The emission stop threshold value can be set more specifically in the electronic cassette 13 in the second AEC mode, as compared with the case of executing the first AEC mode using the emission stop threshold value preset in the X-ray generating apparatus 2a.

To be more specific, according to the imaging conditions preset in the source control device 11, only one imaging condition (including the emission stop threshold value) is set relative to one tube voltage, which depends on the body part to be imaged, as shown in Fig. 2. On the other hand, according to the imaging conditions settable in the electronic cassette 13, a plurality of imaging conditions are settable relative to one tube voltage, as shown in Fig. 6. Taking Fig. 6 as an example, emission stop threshold values (S values) different in accordance with an imaging direction (PA, AP) and the like are settable relative to one tube voltage (120 kV) corresponding to the chest radiography. The information settable to the electronic cassette 13 is stored in a storage device 87 of the console 14.

Note that, an S value is used as the emission stop threshold value set in the electronic cassette 13. The S value, which is obtained by a histogram analysis of the X-ray image data, is a representative index value of a radiation dose, similarly to an EI value and a REX value. Although the S value differs from a value representing a radiation dose itself just as with the emission stop threshold value preset in the source control device 11, the S value can be converted into a dose value similar to the emission stop threshold value present in the source control device 11.

The emission stop threshold value set in the electronic cassette 13 is set as a value to be compared with the new AEC detection signal before being corrected by the corrector 76. The new AEC detection signal inputted to the comparator 78 has been corrected by the corrector 76, as described above, so the emission stop threshold value that is set to be compared with the uncorrected new AEC detection signal needs to be converted. The threshold value generator 79 replaces the emission stop threshold value set in the electronic cassette 13 with a value comparable with the corrected new AEC detection signal.

More specifically, the threshold value generator 79 converts the emission stop threshold value set as a form of the S value to the electronic cassette 13 into a form of the dose value. Then, the converted dose value is multiplied by a ratio between an output value of the previous AEC detection signal and an output value of the uncorrected new AEC detection signal, to obtain the emission stop threshold value comparable to the corrected new AEC detection signal. Taking a case as an example where the emission stop threshold value converted from the S value set in the electronic cassette 13 is "6" , and a ratio between the output value (4) of the previous AEC detection signal and the output value (5) of the uncorrected new AEC detection signal is "4/5 (0.8)", the emission stop threshold value to be compared with the corrected new AEC detection signal is calculated by 6x0.8=4.8. The ratio between the output value of the previous AEC detection signal and the output value of the uncorrected new AEC detection signal is obtained from source information 99 described later on.

Note that, the replacement of the emission stop threshold value, as described above, is required in this embodiment, because the new AEC detection signal after being corrected by the corrector 76 is inputted to the comparator 78 in the second AEC mode. However, inputting the new AEC detection signal before the correction to the comparator 78 eliminates the need for replacing the emission stop threshold value.

The communication unit 40 includes a detection signal I/F 80 and an emission signal I/F 81, in addition to the antenna 37 and the socket 39 described above. The detection signal I/F 80 is wirelessly connected to the detection signal I/F 26 of the source control device 11, and the emission signal I/F 81 is wirelessly connected to the emission signal I/F 27 of the source control device 11. The communication between the detection signal I/Fs 26 and 80 and between the emission signal I/Fs 27 and 81 adopts a relatively high speed wireless communication method, for example, an optical wireless communication, notably infrared communication such as IrDA. The detection signal I/F 26 and the emission signal I/F 27 function as a high speed communication unit. The detection signal I/F 80 and the emission signal I/F 81 function as a high speed communication unit.

To the detection signal I/F 80, the corrector 76 and the integrator 77 of the AEC unit 67 are connected. The detection signal I/F 80 outputs one of an output from the corrector 76 i.e. the new AEC detection signal and an output of the integrator 77 i.e. the integrated value of the new AEC detection signal. The emission signal I/F 81 performs the transmission and reception of the start synchronization signal (emission start request signal and the emission permission signal), the output of the comparator 78 i.e. the transmission of the emission stop signal. In the execution of the AEC, the detection signal I/F 80 is used in the first AEC mode, and the emission signal I/F 81 is used in the second AEC mode. The emission signal I/F 81 is used for the transmission and reception of the start synchronization signal in both of the first AEC mode and the second AEC mode.

The console 14 is communicatably connected to the electronic cassette 13 in a wired or wireless method, to control the operation of the electronic cassette 13. To be more specific, the console 14 transmits the imaging condition to the electronic cassette 13 to set a signal processing condition (including a gain of the amplifier for multiplying a voltage corresponding to the accumulated signal charge) of the FPD 35. Additionally, the console 14 turns on and off the electronic cassette 13, and puts the electronic cassette 13 into a power saving mode, an exposure preparation mode, and the like.

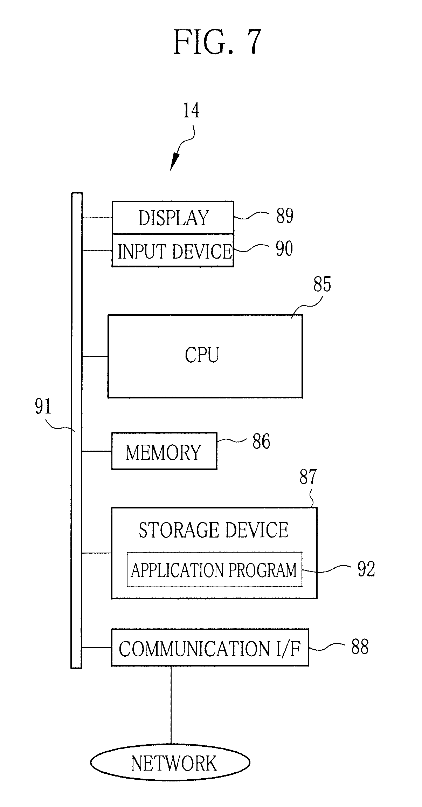

The console 14 applies various types of image processes including an offset correction, a gain correction, a defect correction, and the like to the X-ray image data transmitted from the electronic cassette 13. In the defect correction, pixel values of the row having the detection pixel 65 are interpolated using the pixel values of the adjacent row without having the detection pixel 65. The X-ray image after subjected to the image processes is displayed on a display 89 (see Fig. 7) of the console 14, and its data is stored to the storage device 87 and a memory 86 (both are shown in Fig. 7) of the console 14, or a data storage such as an image storage server connected to the console 14 through a network.

The console 14 receives an input of an examination order including information about sex and age of a patient, a body part to be imaged, and an examination purpose, and displays the examination order on the display 89. The examination order is inputted from an external system e.g. HIS (hospital information system) or RIS (radiography information system) that manages patient data and examination data related to radiography, or inputted manually by the operator. The examination order includes the body part to be imaged e.g. head, chest, abdomen, and the like, and an imaging direction e.g. anterior, medial, diagonal, PA (X-rays are applied from a posterior direction), and AP (X-rays are applied from an anterior direction). The operator confirms the contents of the examination order on the display 89, and inputs the imaging condition corresponding to the contents through an operation screen of the console 14.

In Fig. 7, the console 14 is composed of a computer having a CPU 85, the memory 86, the storage device 87, a communication I/F 88, the display 89, and an input device 90. These components are connected to each other via a data bus 91.

The storage device 87 is a hard disk drive (HDD), for example. The storage device 87 stores a control program and an application program (hereafter called AP) 92. The AP 92 is a program to make the console 14 execute various functions related to the radiography including a display process of the examination order and the X-ray image, the image process of the X-ray image, a setup of the imaging condition, and the like.

The memory 86 is a work memory used when the CPU 85 executes a process. The CPU 85 loads the control program stored on the storage device 87 into the memory 86, and performs a process in accordance with the program for centralized control of each part of the computer. The communication I/F 88 is a network interface for performing wireless or wired transmission control from/to an external device such as the RIS, the HIS, the image storage server, or the electronic cassette 13. The communication I/F 88 corresponds to a low speed wireless communication unit and a low speed wired communication unit. The input device 90 includes a keyboard and a mouse, or a touch panel integrated with the display 89.

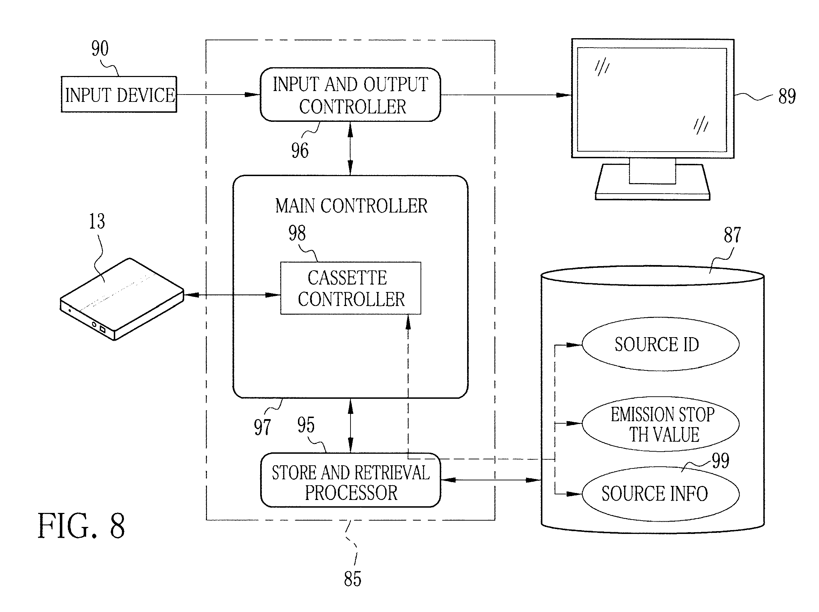

In Fig. 8, by running the AP 92, the CPU 85 of the console 14 functions as a store and retrieval processor 95, an input and output controller 96, and a main controller 97. The store and retrieval processor 95 performs a storing process of various types of data to the storage device 87, and a retrieval process of the various types of data stored in the storage device 87. The input and output controller 96 reads out drawing data from the storage device 87 in response to an operation on the input device 90, and outputs to the display 89 various operation screens of GUIs based on the read drawing data. The input and output controller 96 receives an input of operation commands from the input device 90 through the operation screen. The main controller 97 has a cassette controller 98 for controlling the operation of the electronic cassette 13, and performs centralized control of each part of the console 14.

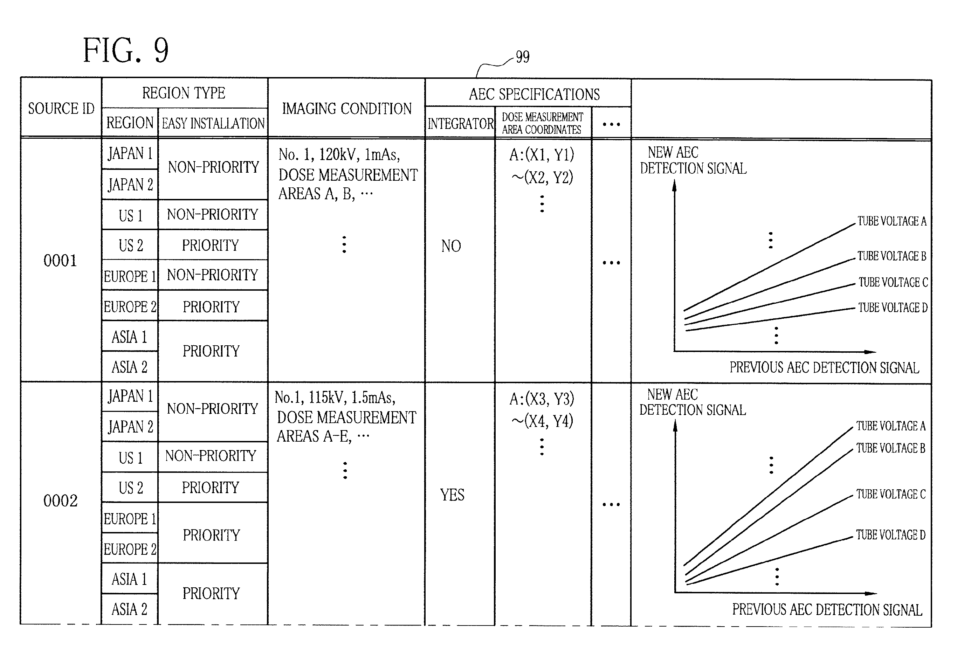

The storage device 87 stores source information 99 as shown in Fig. 9. The source information 99 is referred to determine a setting and a connection method of the X-ray imaging apparatus 2b, in the combined use of the X-ray generating apparatus 2a having the X-ray source 10 and the X-ray imaging apparatus 2b having the electronic cassette 13. The source information 99 includes specifications of the X-ray generating apparatus 2a, specifications of the previous AEC sensor 25 that has been used together with the X-ray generating apparatus 2a from before installation of the X-ray imaging apparatus 2a, the imaging condition preset in the X-ray generating apparatus 2a, and a region type by which a connection type considered to be appropriate in accordance with a geographic region where the X-ray generating apparatus 2a is installed is specified, which are stored on a source ID basis. The source ID represents the model of the X-ray generating apparatus 2a.

The region type is information representing that which form is suited for use in the connection between the X-ray imaging apparatus 2b and the X-ray generating apparatus 2a in accordance with the geographic region (region such as Japan, North America, Europe, and Asia) where the X-ray generating apparatus 2a has already been present and the X-ray imaging apparatus 2b is newly installed. In the region type, there are two forms in the connection, that is, a connection method of giving a priority to ease of installation (easy installation priority type) with a penalty in performance (e.g. performance contributing to improvement in X-ray image quality) to some extent, and a connection method of making full use of the performance of the X-ray imaging apparatus 2b (easy installation non-priority type) with a penalty in the ease of installation to some extent. Which connection method to use is set in advance from region to region, and therefore the connection method is chosen in accordance with the geographic region where the X-ray imaging apparatus 2b is installed. According to the set connection method, the electronic cassette 13 is put into one of the first AEC mode and the second AEC mode.

In a case where a medical facility having the X-ray generating apparatus 2a intends to be newly equipped with the X-ray imaging apparatus 2b, a serviceman is in charge of the installation of the X-ray imaging apparatus 2b, including an initial setting of the X-ray imaging apparatus 2b and a connection to the X-ray generating apparatus 2a. However, depending on an geographic area, it may be difficult to make arrangements for a skilled serviceman.

As described above, the electronic cassette 13 does not need to be connected to the detection signal I/F 26 of the source control device 11 in the second AEC mode. However, the previous AEC sensor 25 used conventionally is connected to the detection signal I/F 26. Therefore, a serviceman who does not have enough knowledge of the connection method of the electronic cassette 13 may mistakenly connect the electronic cassette 13 to the detection signal I/F 26 to which the previous AEC sensor 25 has been connected in the instance of detaching the previous AEC sensor 25 from the X-ray generating apparatus 2a and connecting the electronic cassette 13 thereto, though the electronic cassette 13 has to be connected to the emission signal I/F 27 in actual fact.

Placing a priority on ease of installation, the detection signal I/F 26 is preferably used, because the electronic cassette 13 is connected in a manner similar to the connection of the conventionally used previous AEC sensor 25 and the skill of the serviceman has no effect on a connection result. This is because the electronic cassette 13 has the first AEC mode in which the detection signal I/F 26 is used as in the case of the previous AEC sensor 25. The electronic cassette 13 is connected to the detection signal I/F 26 and operated in the first AEC mode, in the easy installation priority type.

On the other hand, as explained in Figs. 2 and 6 with comparison, the number of the imaging conditions (including the emission stop threshold values) preset in the source control device 11 is limited, and hence a precise setting cannot be made in performing the AEC by the source control device 11. In performing the AEC by the electronic cassette 13, it is possible to make a more precise setting (including the emission stop threshold value). Therefore, the second AEC mode in which the electronic cassette 13 performs the AEC based on a precise imaging condition is superior to the first AEC mode using the emission stop threshold value preset in the source control device 11 in the X-ray image quality and the like, except for the ease of installation.

As described above, the electronic cassette 13 can be selectively switchable between the two types, that is, the connection method of putting a priority on ease of installation that has a high degree of commonality to the connection method of the previous AEC sensor 25 (first AEC mode is chosen) and the connection method of putting a higher priority on improvement in the image quality than the ease of installation (second AEC mode is chosen). Note that, in this embodiment, these types are related to the geographic regions and set as the region types, but one of the easy installation priority type (first AEC mode) and the easy installation non-priority type (second AEC mode) may be selectable in accordance with a user's preference irrespective of the geographic region.

The imaging conditions of the source information 99 are the same as those stored in the source control device of each X-ray source, other than the emission stop threshold values that can be adjusted by the operator. The AEC specifications have items of the presence or absence of the integrator for integrating the AEC detection signal, the positions of the dose measurement areas in the previous AEC sensor 25 (X and Y coordinates of two points on a diagonal line if the dose measurement area is rectangular), which value to output out of a value of each individual dose measurement area, a sum value of the dose measurement areas, and an average value of the dose measurement areas in the previous AEC sensor 25 (not shown).

The X and Y coordinates correspond to the position of the pixels 45 (including the detection pixels 65) of the electronic cassette 13 in the imaging surface 36. An X axis extends to a direction parallel to the scan line 51. A Y axis extends to a direction parallel to the signal line 52. The coordinates of the top left pixel 45 are designated as an origin point (0, 0). Information about the positions of the dose measurement areas is referred by the dose measurement area selector 75 to determine which output to select, out of outputs of the detection pixels 65 in the electronic cassette 13. The dose measurement area selector 75 selects the new AEC detection signal of the detection pixel 65 that is situated in an area corresponding to the dose measurement area of the previous AEC sensor 25, out of the AEC detection signals of the plurality of detection pixels 65 based on the information on the dose measurement areas.

Note that, there is an imaging stand or table on which the electronic cassette 13 can be mounted in an orientation rotated by 90° such as a portrait orientation and a landscape orientation. In the case of using such an imaging stand or table, if the dose measurement area selector 75 selects the dose measurement area by accepting the information on the dose measurement areas of the previous AEC sensor 25 without questioning, as described above, the dose measurement area is selected in a completely different position depending on the orientation of the electronic cassette 13. To prevent this, as described in Japanese Patent Laid-Open Publication No. 2011-067314, for example, it is preferable that a mounted orientation of the electronic cassette on the imaging stand or table is detected using a photosensor or the like and the dose measurement area selector 75 selects the dose measurement area based on information of the detection result.

More specifically, when the information on the positions of the dose measurement areas in the previous AEC sensor 25 corresponds to the portrait orientation and the electronic cassette 13 is mounted in the landscape orientation, the information (coordinates) on the dose measurement areas in the previous AEC sensor 25 is used after being rotated by 90° or 270° with respect to the center of the imaging field of the cassette. Otherwise, the source information 99 has information on the positions of the dose measurement areas in the previous AEC sensor 25 corresponding to the portrait orientation and the landscape orientation in advance, and information to be used may be selected in accordance with the detection result of the mounted orientation of the cassette.

The source information 99 also includes correction information. The correction information is referred by the corrector 76 and used for making the output value of the new AEC detection signal correspond to the output value of the previous AEC detection signal. Also, the correction information is referred by the threshold value generator 79 in the execution of the second AEC mode. The threshold value generator 79 calculates the ratio between the output value of the new AEC detection signal and the output value of the previous AEC detection signal based on the correction information. The calculated ratio is used in replacing the emission stop threshold value. The correction information represents the correlation between the new AEC detection signal and the previous AEC detection signal of each X-ray source on a tube voltage basis and stored in a form of a data table or an arithmetic expression.

The previous AEC sensor 25 and the detection pixels 65 of the electronic cassette 13 are different in an installation state and the like, in addition to a sensitivity property. Thus, a difference occurs between the output value of the previous AEC sensor 25 and the output value of the detection pixels 65 even if the same X-ray dose is applied.

Since the previous AEC sensor 25 is used in a state of being put in front of the imaging surface of the cassette, the previous AEC sensor 25 itself causes reduction in the amount of the X-rays to be incident from the X-ray source to the imaging surface of the cassette. Therefore, the emission stop threshold value of the previous AEC sensor 25, which is preset in the source control device 11, is determined by adding a radiation dose absorbed by the previous AEC sensor 25 to a radiation dose required for obtaining desired image quality. On the other hand, the electronic cassette 13 uses the detection pixels 65 as the new AEC sensor, and an intermediate member such as the housing of the electronic cassette 13 is disposed between the X-ray source and the new AEC sensor. When the electronic cassette 13 adopts the PSS method in which the scintillator and the FPD 35 are disposed in this order from the X-ray incident side, the scintillator corresponds to the intermediate member too (on the contrary, the scintillator does not correspond to the intermediate member in the ISS method). If a grid for eliminating the X-rays scattered inside the object is provided between the X-ray source 10 and the electronic cassette 13 in the introduction of the electronic cassette 13, the grid corresponds to the intermediate member too. In the case of using the detection pixels 65 of the electronic cassette 13 for the AEC, instead of the previous AEC sensor 25, if an application of a radiation dose causes the previous AEC detection signal of a value of "1", an application of the same radiation dose may possibly cause the new AEC detection signal of "0.8" due to the disposition of the intermediate member.

Furthermore, an output range may differ between the previous AEC detection signal and the new AEC detection signal, such that the previous AEC detection signal is represented in a range having a minimum value of -5 V and a maximum value of 5 V, while the new AEC detection signal is represented in a range having a minimum value of 0 mV and a maximum value of 5 mV. Thus, in the case of using the electronic cassette 13, it is required to correct a deviation between the previous AEC detection signal and the new AEC detection signal caused by the existence of the intermediate member and the difference in the output range. The correction information facilitates eliminating the deviation between the output value of the previous AEC detection signal and the output value of the new AEC detection signal in the application of the same X-ray dose.

The correction information is calculated in advance by experiment and simulation in consideration of the structure of the previous AEC sensor 25 and the structure of the electronic cassette 13 (the PSS method or the ISS method, the presence or absence of the scintillator and a material of the scintillator if it is present, the presence or absence of the grid and a material of the grid if it is present, and the like). Note that, the presence or absence of the scintillator is obtained from the specifications of the electronic cassette 13, i.e. the PSS method or the ISS method. The presence or absence of the grid is chosen through a GUI displayed on the display 89 of the console 14. Irrespective of the intermediate member, the previous and new AEC sensors have different X-ray detection principles, so a detection value is different therebetween even if the same radiation dose is applied. The above experiment and simulation also eliminate a deviation in the detection value due to the difference in the detection principles.

The source information 99 is setting information employed in using the electronic cassette 13 together with the X-ray generating apparatus 2a. Thus, the source information 99 is produced on a model-by-model basis of the electronic cassette 13. The source information 99, which is produced on a model-by-model basis of the electronic cassette 13, is updated anytime to the latest information provided through the network, whenever a new product of the X-ray source is released. Instead of the automatic update, X-ray source information that is possibly used in the system may be obtained from a manufacturer and inputted manually through the input device 90.

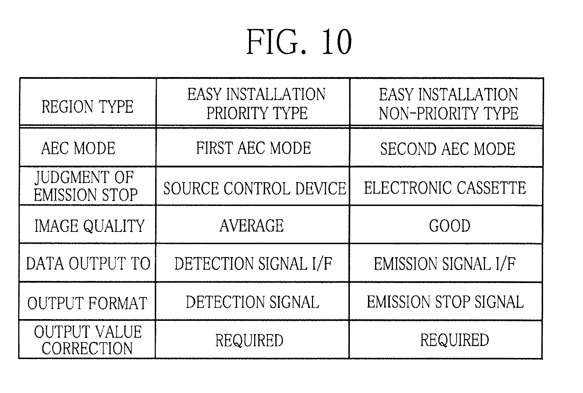

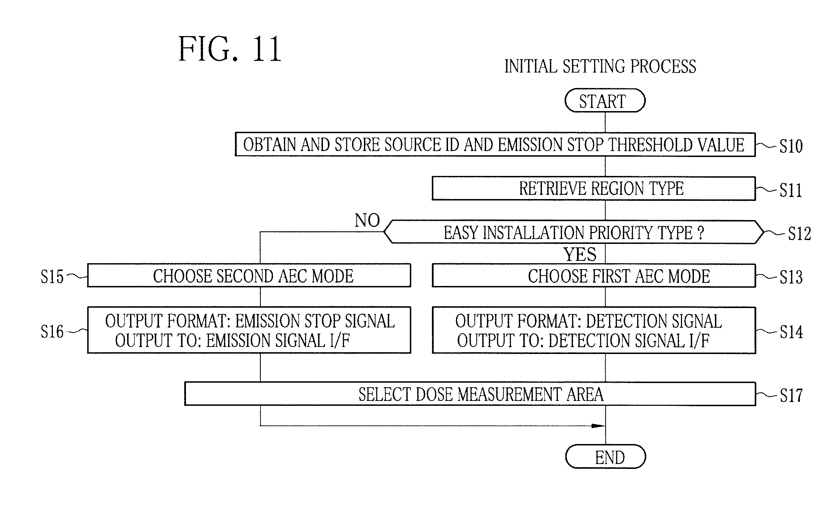

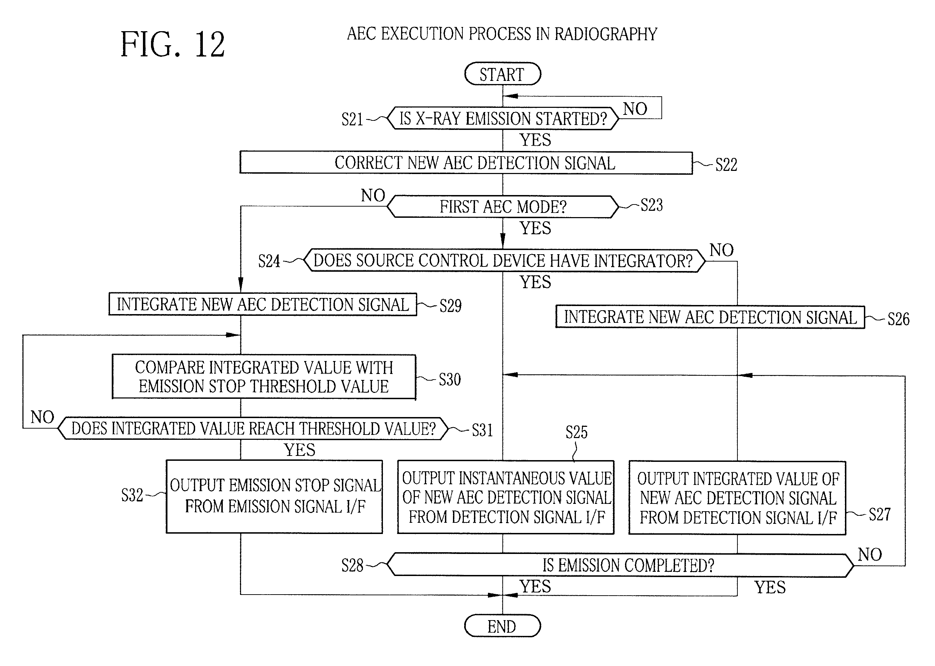

A table of Fig. 10 shows the setting and the like of the electronic cassette 13 in each region type, that is, in each of the easy installation priority type (first AEC mode) and the easy installation non-priority type (second AEC mode). The operation of the above structure will be hereinafter explained with referring to the table of Fig. 10, a flowchart of an initial setting process shown in Fig. 11, a flowchart of an AEC execution process in radiography shown in Fig. 12, and Figs. 13 to 15 representing operation states of the communication unit 40 and the AEC unit 67.

A case of newly introducing the X-ray imaging apparatus 2b having the electronic cassette 13 and the console 14 into the X-ray imaging system 2, instead of the film cassette, the IP cassette, and the previous AEC sensor 25 that are conventionally used, will be described as an example.

In installing the X-ray imaging apparatus 2b, the electronic cassette 13 and the console 14 are wirelessly connected using the antenna 37 of the electronic cassette 13. Then, the communication I/F 88 of the console 14 is connected to the communication I/F 22 of the source control device 11 through a network such as a LAN.

As shown in a step 10 (S10) of Fig. 11 representing the initial setting process, upon establishing communication between the console 14 and the source control device 11, the store and retrieval processor 95 obtains information of the source ID and the emission stop threshold value preset in the source control device 11 through the communication I/F 22 of the source control device 11, and stores the information to the storage device 87 (see Fig. 8 too).

The store and retrieval processor 95 retrieves and extracts a type that corresponds to the source ID received by the source control device 11 and the geographic region set in advance in shipping (the region to which the X-ray imaging apparatus 2b is to be installed) from the item of the region type of the source information 99 (S11). The imaging condition, the AEC specifications, and the correction information corresponding to the source ID are extracted from the source information 99. This information extracted by the store and retrieval processor 95 is provided from the cassette controller 98 to the electronic cassette 13 together with the information of the emission stop threshold value. The information extracted by the store and retrieval processor 95 is also displayed on the display 89 of the console 14.

The serviceman in charge of the installation of the X-ray imaging apparatus 2b checks the display on the display 89, and establishes physical connection between the electronic cassette 13 and the X-ray generating apparatus 2a in accordance with the geographic region set in shipping the X-ray imaging apparatus 2. In the region where the ease of installation is prioritized, the emission signal I/F 27 of the source control device 11 is connected to the emission signal I/F 81 of the electronic cassette 13, and the detection signal I/F 26 of the source control device 11 is connected to the detection signal I/F 80 of the electronic cassette 13 as the connection I/F for the AEC signal. The detection signal I/F 26 has been used with the previous AEC sensor 25, so the serviceman is hardly confused at the connection method even if the serviceman does not have enough knowledge. In the region where the ease of installation is not prioritized, only the emission signal I/F 27 and the emission signal I/F 81 are connected each other, while the detection signal I/Fs 26 and 80 are not used.

The controller 41 of the electronic cassette 13 chooses the AEC mode to be executed by the electronic cassette 13 based on information of the region type provided by the console 14, from between the first AEC mode and the second AEC mode. In accordance with the chosen mode, an output destination and the output format of the AEC signal are determined.

To be more specific, in a case where the region type is the easy installation priority type (YES in S12), the first AEC mode is chosen (S13). Thus, the detection signal I/F 80 is designated as the output destination of the communication unit 40, and the detection signal (new AEC detection signal) is designated as the output format (S14). In a case where the region type is the easy installation non-priority type (NO in S12), the second AEC mode is chosen (S15). The emission signal I/F 81 is designated as the output destination, and the emission stop signal is designated as the output format (S16). In the case of choosing the first AEC mode, the output format is chosen in further detail depending on the presence or absence of an integrator usable in the AEC and information about which value to output out of the value of each individual dose measurement area, the sum value of the dose measurement areas, and the average value of the dose measurement areas.

The dose measurement area selector 75 selects the new AEC detection signal of the detection pixel 65 that is present in the area corresponding to the dose measurement area of the previous AEC sensor 25, out of the new AEC detection signals of the plurality of detection pixels 65 outputted from the A/D 62, based on the information on the position of the dose measurement area of the previous AEC sensor 25 provided by the console 14. The dose measurement area selector 75 outputs the selected new AEC detection signal to the corrector 76 (S17). Taking the case of a source ID "0001" in this embodiment as an example, the dose measurement area selector 75 selects the new AEC detection signals of the detection pixels 65 that are present within frames A' to C' as shown in Fig. 4, corresponding to the dose measurement areas

A to C. Then, the initial setting is completed.

In the radiography using the X-ray imaging system 2 after the completion of the initial setting, the imaging condition is set based on the examination order. Upon inputting the warm-up start signal from the emission switch 12, the source control device 11 sends the emission start request signal to the electronic cassette 13 through the emission signal I/F 27. The electronic cassette 13 performs the preparation process, and sends the emission permission signal to the source control device 11 as soon as the electronic cassette 13 is ready for receiving the X-ray emission. Upon receiving the emission permission signal, the source control device 11 makes the X-ray source 10 start the X-ray emission.

As shown in Fig. 12, upon sending the emission permission signal, the electronic cassette 13 judges that the X-ray emission has been started and makes the detection pixels 65 start the dose detection operation (YES in S21).

Upon starting the dose detection operation, the new AEC detection signals are recorded from the detection pixels 65 to the memory 42. In the AEC unit 67, the dose measurement area selector 75 reads out the selected new AEC detection signal from the memory 42. The corrector 76 converts the new AEC detection signal inputted from the dose measurement area selector 75 into the detection signal based on the correction information corresponding to the source ID of the X-ray generating apparatus 2a (S22). The corrector 76 calculates the sum value, the average value, or the like of the detection signals if necessary, based on the information about which value to output out of the value of each individual dose measurement area, the sum value of the dose measurement areas, and the average value of the dose measurement areas. The selection of the dose measurement area and the correction, as described above, are necessarily carried out irrespective of the selected AEC mode (see Fig. 10 too).

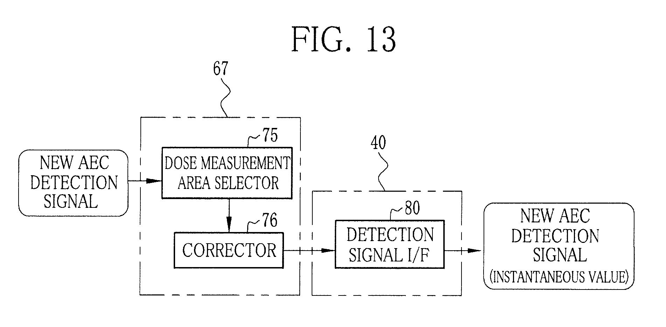

In a case where the first AEC mode is chosen in the initial setting (YES in S23) and the source control device 11 is judged to have an integrator based on the information on the presence or absence of the integrator usable in the AEC (YES in S24), the controller 41 of the electronic cassette 13 transmits the instantaneous value of the new AEC detection signal outputted from the corrector 76 at constant transmission intervals through the detection signal I/F 80 to the detection signal I/F 26 of the source control device 11 (S25). In this case, in the AEC unit 67, only the dose measurement area selector 75 and the corrector 76 function as shown in Fig. 13.

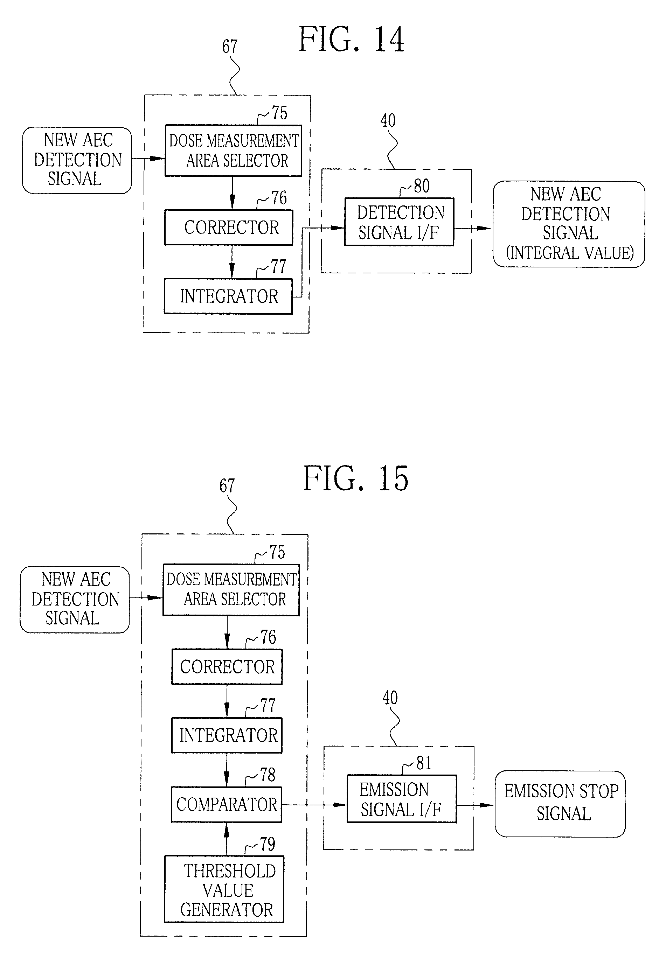

On the other hand, when the first AEC mode is chosen and the source control device 11 has no integrator (NO in S24), the corrector 76 outputs the new AEC detection signal to the integrator 77. The integrator 77 integrates the new AEC detection signal (S26). The integrated value of the new AEC detection signal is transmitted at constant transmission intervals from the integrator 77 through the detection signal I/F 80 to the detection signal I/F 26 of the source control device 11 (S27). The instantaneous value or the integrated value of the detection signal is continuously transmitted until electronic cassette 13 receives an emission end signal from the source control device 11 (YES in S28). In this case, as shown in Fig. 14, the dose measurement area selector 75, the corrector 76, and the integrator 77 function in the AEC unit 67.

In the first AEC mode, the instantaneous value of the integrated value of the new AEC detection signal is transmitted from the electronic cassette 13 to the source control device 11. The source control device 11, which receives the instantaneous value or the integrated value of the new AEC detection signal, makes a judgment on the stop of X-ray emission. Just as in the case of using the previous AEC sensor 25, the judgment of the stop of X-ray emission is made by comparison of the integrated value of the new AEC detection signal with the emission stop threshold value. After the completion of the X-ray emission, the electronic cassette 13 reads out the X-ray image from the FPD 35, and transmits the X-ray image data to the console 14 through the antenna 37.

When the second AEC mode is chosen (NO in S23), the comparator 78 and the threshold value generator 79 function in addition to above, as shown in Fig. 15. First, just as in a case where the source control device 11 has no integrator in the first AEC mode, the corrector 76 outputs the new AEC detection signal to the integrator 77, and the integrator 77 integrates the new AEC detection signal (S29).

The comparator 78 compares the integrated value of the new AEC detection signal from the integrator 77 with the emission stop threshold value from the threshold value generator 79 (S30) . As soon as the integrated value reaches the threshold value (YES in S31), the emission stop signal is outputted. The emission stop signal outputted from the comparator 78 is transmitted through the emission signal I/F 81 to the emission signal I/F 27 of the source control device 11 (S32). Upon receiving the emission stop signal, the source control device 11 stops the X-ray emission. Also in the second AEC mode, upon the completion of the X-ray emission, the electronic cassette 13 reads out the X-ray image from the FPD 35, and transmits the X-ray image data to the console 14 through the antenna 35.

In the second AEC mode, the corrector 76 corrects the new AEC detection signal from the detection pixel 65 into the detection signal corresponding to the previous AEC detection signal. The corrected new AEC detection signal is compared with the emission stop threshold value to make a judgment of the stop of X-ray emission. In other words, the electronic cassette 13 carries out exactly the same process in the second AEC mode as the process of the AEC that the controller 21 of the source control device 11 carries out in the radiography using the previous AEC sensor 25 or in the first AEC mode. However, the emission stop threshold value varies in accordance with each of the plurality of imaging conditions, so it is possible to realize the precise AEC, as compared with the AEC performed by the source control device 11. Therefore, the image quality is improved in the second AEC mode, as compared with the image quality in the first AEC mode.

The electronic cassette 13 is switchable between the first AEC mode and the second AEC mode, which have different connection methods to the X-ray generating apparatus 2a, in accordance with the region type, that is, the easy installation priority type or the easy installation non-priority type. Therefore, it is possible to flexibly meet a situation of a site where the X-ray imaging system 2 is installed.

Since the electronic cassette 13 has the first AEC mode in which the new AEC detection signal, being equivalent to the previous AEC detection signal outputted from the previous AEC sensor 25, is outputted to the source control device 11, the source control device 11 can use the electronic cassette 13 just as in the case of using the previous AEC sensor 25 without correcting the preset emission stop threshold value and changing a preset judging process. When the X-ray generating apparatus and the X-ray imaging apparatus are made by different makers, correcting the emission stop threshold value of the source control device 11 requires a serviceman of the source maker on site and hence expenses much time and effort. The present invention, however, facilitates less burdensome because the correction is completed just in the electronic cassette 13, and this becomes a sales point in introducing the new system. Furthermore, it is possible to inherit a tendency of an operator and a policy of a hospital, e.g. reducing radiation exposure of a patient by using a low radiation dose or increasing an X-ray image density by using a high radiation dose.

Also, the dose measurement area selector 75 selects the detection pixel 65 such that the electronic cassette 13 has the same dose measurement area as the dose measurement area of the previous AEC sensor 25, so the AEC can be carried out in a like manner as previous.

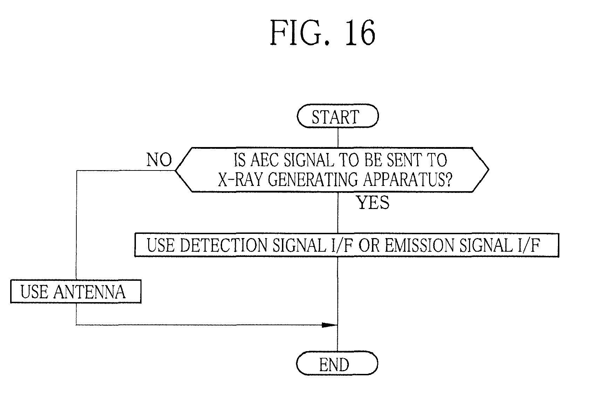

As shown in a flowchart of Fig. 16, the electronic cassette 13 uses the detection signal I/F 80 and the emission signal I/F 81, being the high speed communication unit, as the communication unit in both of the first AEC mode and the second AEC mode, in a case where a signal is to be sent to the X-ray generating apparatus 2a, just as in the case of sending the AEC signal (new AEC detection signal and the emission stop signal). Thus, the AEC signal can be sent quickly in the AEC process, which is carried out within extremely short emission time. Accordingly, this prevents a delay in timing of the stop of X-ray emission and hence unnecessary radiation exposure of a patient.

On the other hand, the electronic cassette uses the antenna, being the low speed wireless communication unit, as the communication unit, in a case where a signal e.g. the X-ray image is to be sent to the console 14. The electronic cassette 13 and the console 14 are connected by a wireless method without a cable, and therefore the handleability and the portability of the electronic cassette 13 and the console 14 are ensured. The electronic cassette 13 and the console 14 are sometimes situated away from each other, such that the electronic cassette 13 is disposed in an examination room while the console 14 is installed in an operators room. Thus, connecting the electronic cassette 13 and the console 14 without a cable is highly effective. Especially, the cableless connection produces a beneficial effect in using the electronic cassette 13 in a state of being put on a bed or held by the object himself/herself.

In addition to the X-ray image, the imaging condition, the life check signal, and the like are communicated between the electronic cassette 13 and the console 14. Transmission of these signals requires less rapidity than the AEC signal, and therefore uses a lower speed communication unit than a communication speed of the communication unit of the AEC signal.

In this embodiment, since the battery 38 supplies the drive power of the electronic cassette 13, the power cable for feeding the electric power to the electronic cassette 13 is unnecessary. Thus, the handleability and the portability of the electronic cassette 13 are further improved.

The battery 38 is taken out of the electronic cassette 13 and set in the cradle 17 for recharging. Thus, it is unnecessary to connect a cable for feeing the electric power to the electronic cassette 13. This further facilitates handling of the electronic cassette 13.

Moreover, in this embodiment, not only the electronic cassette 13 and the console 14, but also the source control device 11 and the electronic cassette 13 are connected by a wireless method. A connection cable is completely eliminated from the electronic cassette 13, so the handleability and the portability of the electronic cassette 13 is further improved. Thus, the electronic cassette 13 can be easily moved among examination rooms.

Note that, in this embodiment, the start synchronization signal (emission start request signal and the emission permission signal) and the emission end signal are communicated between the source control device 11 and the electronic cassette 13 through the emission signal I/Fs 27 and 81. However, the electronic cassette 13 may have the function of detecting the start and end of the X-ray emission by itself. In this case, the communication of the start synchronization signal and the emission end signal becomes unnecessary.

The electronic cassette 13 detects the start and end of the X-ray emission by itself with the use of the new AEC detection signal outputted from the detection pixel 65 provided for the AEC, for example. Upon receiving the X-rays, the detection pixel 65 outputs the new AEC detection signal of a value corresponding to the amount of the X-rays incident thereon. The controller 41 of the electronic cassette 13 compares the instantaneous value of the new AEC detection signal with the predetermined threshold value for judging the start of emission. When the new AEC detection signal exceeds the threshold value, the start of emission is judged and detected. The controller 41 keeps monitoring the new AEC detection signal after the start of emission. When the new AEC detection signal has fallen below the threshold value for judging the end of emission, the end of emission is judged and detected.

Providing the self detection function of the start and end of emission, as described above, negates the need for communication of the start synchronization signal and the emission stop signal between the electronic cassette 13 and the source control device 11. Thus, in the first AEC mode in which the AEC signal is outputted to the detection signal I/F 26, the detection signal I/Fs 26 and 80 are connected each other, and the emission signal I/Fs 27 and 81 are connected each other in the above embodiment. Out of these connections, the connection between the emission signal I/Fs 27 and 81 becomes unnecessary. In other words, only the connection between the detection signal I/Fs 26 and 80 is required in the first AEC mode. In the second AEC mode, since the AEC signal (emission stop signal) is outputted to the emission signal I/F 27, the emission signal I/Fs 27 and 81 are connected as described above.

### Second Embodiment

In the above first embodiment, the optical wireless communications is used as an example of high speed communications of the detection signal or the emission stop signal for the AEC between the source control device 11 and the electronic cassette 13, and the wireless LAN or the like is used as an example of low speed wireless communications of various types of information and signals other than the detection signal and the emission stop signal for the AEC between the electronic cassette 13 and the console 14. However, ad-hoc communications may be used in the former communications, and infrastructure communications may be used in the latter communications.

The ad-hoc communications is a method in which wireless communication devices (the source control device 11 and the electronic cassette 13) have a routing function of a wireless communication channel and each wireless communication device performs communication on an autonomous basis. Thus, the ad-hoc communications is established without mediation of a relay device having the routing function such as a switching hub or a router between the wireless communication devices. The ad-hoc communications is a so-called specific line method used only in the X-ray imaging system 2. Thus, the ad-hoc communications hardly causes a delay (time lag) in data communication, and average delay time in the data communication becomes small.

On the contrary, the infrastructure communications is a method for establishing communication with mediation of a relay device having a routing function. In the infrastructure communications, the relay device having the routing function is a component of a network such as a hospital LAN that performs communication of medical equipment including the X-ray imaging system 2 and other devices, and therefore used in data communication of signals and data sent and received by the devices other than the X-ray imaging system 2, such as an electronic medical chart, a medical report, and account data. Thus, the infrastructure communications more easily causes a delay (time lag) in data communication than the ad-hoc communications. The ad-hoc communications has higher communication speed than the infrastructure communications.

The ad-hoc communications includes a wireless method using a radio wave, in addition to the optical wireless communication method as described above. In the ad-hoc communications, it is preferable that the source control device 11 and the electronic cassette 13 establish direct communication without mediation of any relay device. Note that, in the ad-hoc communications, a relay device having no routing function, e.g. a repeater having the function of just transferring a received signal, an amplifier for amplifying a signal in the middle of transfer for restraining attenuation of the signal, and the like may be provided. This is because such a relay device does not cause much data delay.

Note that, if a communication speed is the same in the specifications, an actual communication speed differs in reality depending on average delay time in the data communication. In other words, in the present invention, a high communication speed and a low communication speed are defined in consideration of not only the communication speed itself but also the average delay time in the data communication. Therefore, "the high speed communication unit" of the present invention includes a device that has relatively small average delay time in the data communication, and "the low speed wireless communication unit" includes a device that has relatively large average delay time in the data communication. The ad-hoc communications described above corresponds to high speed communications having relatively small average delay time in the data communication. The infrastructure communications corresponds to low speed communications having relatively large average delay time in the data communication.

The source control device 11 is usually disposed in the examination room. Thus, using the ad-hoc communications in the communication of the detection signal and the emission stop signal for the AEC between the source control device 11 and the electronic cassette 13 allows stable communication, because the source control device 11 and the electronic cassette 13 are close to each other and the radio wave reaches easily, and hence actualizes the high speed communications without the occurrence of a delay in the data communication. The communication of the various types of information and signals other than the detection signal and the emission stop signal for the AEC between the electronic cassette 13 and the console 14, which is usually installed in a room different from the examination room, is performed by the infrastructure communications, so the electronic cassette 13 is connected without a cable and easily handled, just as with the above embodiment.

### Third Embodiment

In the above first and second embodiments, the controller 41 is in charge of controlling the operation of parts including the gate driver 53, the signal processor 54, and the AEC unit 67. The antenna 37 and the socket 39 for making communication with the console 14, and the detection signal I/F 80 and the emission signal I/F 81 for making communication with the source control device 11 are disposed integrally into one communication unit 40. Thus, a process related to the communication with the console 14 conflicts with a process related to the communication with the source control device 11 in the controller 41, or the communication with the console 14 overlaps with the communication with the source control device 11 in the controller 41. As a result, there is a possibility of delaying the transmission timing of the detection signal or the emission stop signal from the detection signal I/F 80 or the emission signal I/F 81.

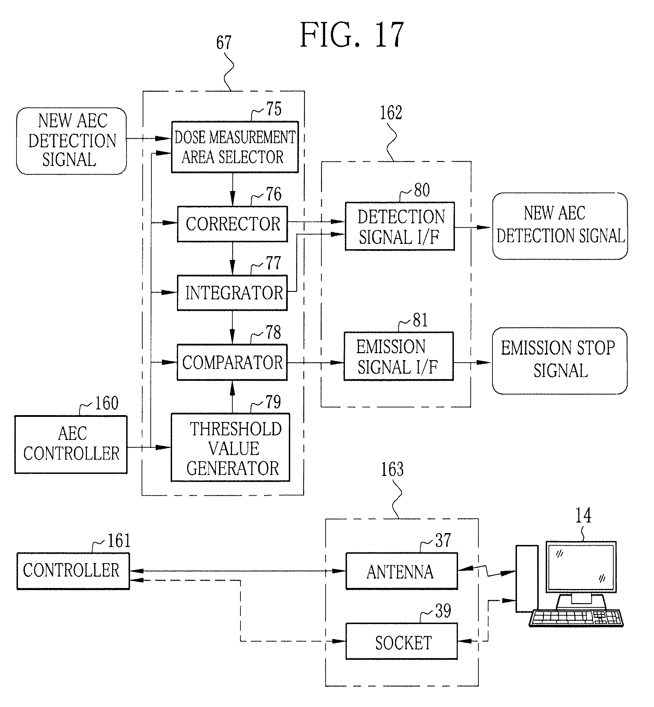

Therefore, in the electronic cassette 13 according to a third embodiment, a control section and a communication section are structured as shown in Fig. 17. Namely, there are provided an AEC controller 160 dedicated to controlling the operation of the AEC unit 67 and another controller 161 for controlling the operation of each part other than the AEC unit 67, which are made of different hardware resources. Also, as for the communication section, there are provided an AEC communication unit 162 having the detection signal I/F 80 and the emission signal I/F 81 and another communication unit 163 having the antenna 37 and the socket 39, which are made of different hardware resources. To be more specific, the AEC controller 160 and the controller 161 are incorporated into different IC chips and operated independently each other. In a like manner, the AEC communication unit 162 and the communication unit 163 are incorporated into different IC chips and operated independently each other.

Since the hardware resources of the control section and the communication section related to the AEC are operated separately from the hardware resources of the other control section and the other communication section, it is possible to prevent the occurrence of the conflict between the process related to the communication with the console 14 and the process related to the communication with the source control device 11 in the control section and the overlap between the communication with the console 14 and the communication with the source control device 11 in the communication section. Thus, the detection signal and the emission stop signal are securely transmitted in desired timing, and the X-ray emission can be stopped precisely.

### Fourth Embodiment

In the above first to third embodiments, the detection signal I/Fs 26 and 80 are wirelessly connected each other, and the emission signal I/Fs 27 and 81 are wirelessly connected each other. However, the detection signal I/Fs 26 and 80 may be connected each other with a cable, and the emission signal I/Fs 27 and 81 may be connected each other with a cable. In this case, for example, an optical fiber cable or the like having a relatively high communication speed is used as the cable. The wired communication consumes less electric power than the wireless communication in general, so it is possible to cut a drain of the battery 38 as compared to the above embodiments.

Since a cable is connected to the electronic cassette 13, the electronic cassette 13 is hard to handle more or less. However, signals transmitted through the cable are limited to some types, i.e. the detection signal and the emission stop signal, so the cable is thinner and more flexible than a cable that is used in connection between the electronic cassette 13 and the console 14 for sending and receiving various types of signals and information. For this reason, the electronic cassette 13 is easy to handle as compared with the case of connecting the electronic cassette 13 and the console 14 with the relatively thick and rigid cable.

Note that, the signals and information other than the AEC signal, such as the image data, may be communicated through a wired connection line of the high speed communications for the AEC signal. In this case, a cable is split in its middle, in such a manner that one is for the AEC signal and the other is for the other signals, and the split cable is connected to the source control device 11 and the console 14. In a case where whether or not the AEC is performed using the detection pixel 65 is set in each of the imaging conditions choosable in the console 14, the controller 41 judges in accordance with the setting that whether or not the imaging condition that executes the AEC is chosen. When the imaging condition that executes the AEC is judged to be chosen, the controller 41 adopts the wireless communication in sending and receiving the image data and the like between the electronic cassette 13 and the console 14, as with the above embodiments. When the imaging condition that does not execute the AEC is judged to be chosen, the controller 41 switches the communication between the electronic cassette 13 and the console 14 to the wired connection line for the AEC. The controller 41 constitutes a judging section and a communication switching section.

To be more specific, when the imaging condition that does not execute the AEC is chosen, the input and output controller 96 of the console 14 displays a message that advises the operator to connect the cable into the socket 39 to establish the wired communication on the display 89. Upon detecting the connection of the cable into the socket 39 to establish the wired communication with the console 14, the controller 41 stops the operation of a function part related to the wireless communication, such as the communication unit 40, and shifts to the wired communication using the cable. In the case of not executing the AEC, no AEC signal is transmitted through the wired connection line for the AEC. Thus, transmitting the signals and information other than the AEC, such as the image data, through the wired connection line for the AEC signal facilitates reducing a drain of the battery 38 due to the wireless communication without a problem of signal collision and the like.

### Fifth Embodiment

In the above first to fourth embodiments, the battery 38 is taken out of the electronic cassette 13 and set in the cradle 17 for recharging, but the present invention is not limited to this. The electronic cassette 13 may have a power receiving function of noncontact power feeding. The battery 38 may be recharged in a state of not being taken out of the electronic cassette 13 with electric power fed from a noncontact power feeding device.

As a method of the noncontact power feeding, there are an electromagnetic induction method, a magnetic resonance method, and an electric field coupling method. Any method is available, but the electric field coupling method has simpler and smaller structure than the other methods, and allows cost reduction and easy control. Also, the electric field coupling method has a relatively high degree of flexibility in positioning and does not require precise positioning, though poor positioning between the power feeding device and a power receiving device (the electronic cassette 13 in this case) significantly reduces electric power transmission efficiency in the other methods. Thus, the electric field coupling method is preferably adopted.

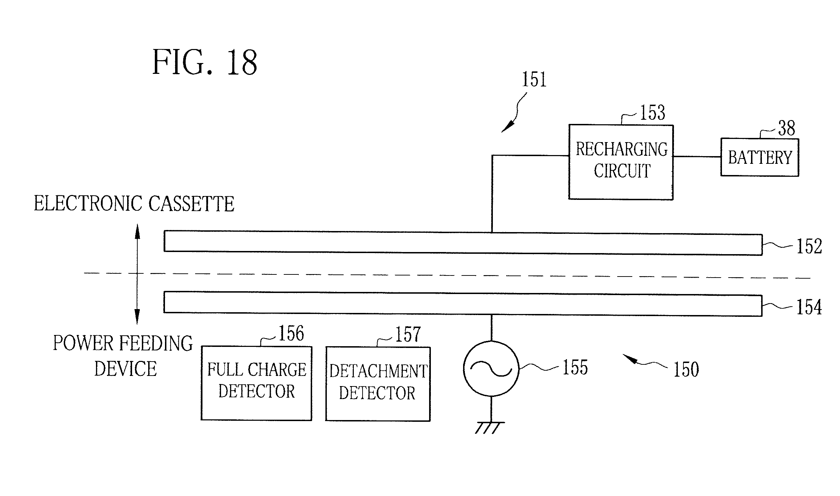

Fig. 18 shows a noncontact power feeding device 150 of the electric field coupling method, as an example. An electronic cassette 151 is provided with a power receiving electrode 152 and a recharging circuit 153. The power receiving electrode 152 is made of metal such as copper or aluminum into a flat plate of approximately the same size and shape as a rear cover of a housing of the electronic cassette 151, so as to be attached to the rear cover, for example.

The power feeding device 150 is contained in the holder 15a of the imaging stand 15 and the holder 16a of the imaging table 16, so that the electronic cassette 151 is fed with power in a state of being mounted on the imaging stand 15 or the imaging table 16. The power feeding device 150 has a power feeding electrode 154. The power feeding electrode 154 is connected to an AC power supply 155. The power feeding electrode 154 is made of the same material as the power receiving electrode 152, and is a flat plate electrode of the same size as the power receiving electrode 152. In setting the electronic cassette 151 in the power feeding device 150, the power feeding electrode 154 is opposed to the power receiving electrode 152 in parallel with leaving space of the order of several millimeters to the power receiving electrode 152. The noncontact power feeding of the electric field coupling method is performed from the power feeding electrode 154 to the power receiving electrode 152. The recharging circuit 153 is constituted of an AC/DC converter (rectifier) and a DC regulator. The recharging circuit 153 converts AC power that is fed from the power feeding electrode 154 and received by the power receiving electrode 152 into DC power, and outputs a voltage suitable for recharging the battery 38.

The power feeding device 150 is provided with a full charge detector 156 and a detachment detector 157. The full charge detector 156 detects whether or not the battery 38 is full. The detachment detector 157 detects detachment of the electronic cassette 151 from the holder 15a or 16a. When the full charge detector 156 detects that the battery 38 is full, or the detachment detector 157 detects the detachment of the electronic cassette 151 from the holder 15a or 16a, a power feeding operation is interrupted by cutting the connection between the power feeding electrode 154 and the AC power supply 155 or stopping the operation of the AC power supply 155.

Recharging the battery 38 with the electric power fed from the noncontact power feeding device 150 eliminates the need for connecting a power feeding cable to the electronic cassette, as with the above embodiments, and hence the electronic cassette is easy to handle.

Note that, the above first to fifth embodiments describe the electronic cassette that can be driven by the integral battery, but the integral battery is not necessarily provided. The electronic cassette may be supplied with power from a utility power supply through a cable. For example, in a case where the electronic cassette is disposed in the examination room and the console is installed in the operators room, the electronic cassette is fed with power from a wall outlet of the utility power supply in the examination room. Eliminating the power cable is preferable as a matter of course in consideration of the handleability and the portability, but the handleability and the portability of the electronic cassette and the console are ensured to some extent because of the cableless connection between the electronic cassette and the console.

### Sixth Embodiment

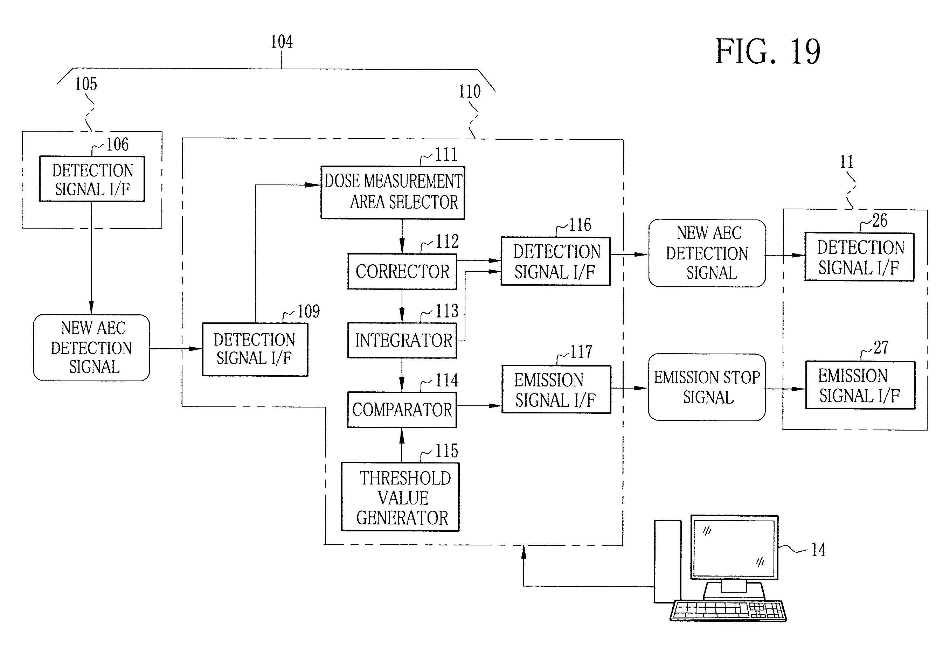

In the first to fifth embodiments, the electronic cassette 13 configured by one housing is provided with all fundamental structures for performing the AEC, which include the AEC unit 67, the detection signal I/F 80, and the emission signal I/F 81. However, in a sixth embodiment as shown in Fig. 19, an electronic cassette 104 is composed of a cassette main body 105 and a supplemental device 110 having a part of a function performing the AEC.

The cassette main body 105 includes the FPD 35 having the detection pixels 65. Furthermore, the cassette main body 105 is provided with a detection signal I/F 106 for outputting the new AEC detection signal from the detection pixel 65 to the supplemental device 110. The supplemental device 110 has all the functions of the AEC unit 67 and the communication unit 40 of Fig. 5. Furthermore, the supplemental device 110 is provided with a detection signal I/F 109, which is connected to the detection signal I/F 106 of the cassette main body 105 to receive the new AEC detection signal. The supplemental device 110 chooses an output format (the new AEC detection signal or the emission stop signal) and an output destination (the detection signal I/F 26 or the emission signal I/F 27) of the AEC signal.

In this case, the supplemental device 110 is connected to the console 14, and receives the region type, the imaging condition, the AEC specifications, the correction information, the emission stop threshold value, and the like of the source information 99 from the console 14. Each part of the supplemental device 110, including a dose measurement area selector 111, a detection signal I/F 116, an emission signal I/F 117, and the like is identical to that of the AEC unit 67 and the communication unit 40 of Fig. 5, though they are referred to by different reference numbers. The supplemental device 110 determines the output destination and the output format in accordance with the region type sent from the console 14, and maintains that state until the X-ray source 10 is exchanged.

Since the functions of the AEC unit 67 and the like are provided in the supplemental device 110, the cassette main body 105 can be small in size and light in weight. In a case where the cassette main body 105 is shared among a plurality of examination rooms of a hospital, if the X-ray generating apparatuses 2a of the examination rooms have different region types, the electronic cassette 13 of the above first embodiment has to change the output destination and the output format from one region type to another. However, since the supplemental device 110 is provided with the changing function, the cassette main body 105 does not have to change the output destination and the output format. A communication method between the detection signal I/F 106 of the cassette main body 105 and the detection signal I/F 109 of the supplemental device 110 may be a wired method or a wireless method. However, the AEC signal that requires the rapidity is communicated between the cassette main body 105 and the supplemental device 110, so the high speed communication unit is adopted, as described as the communication method between the electronic cassette 13 and the source control device 11 in the above first to fifth embodiments.

Note that, it is described in the above first to sixth embodiments that either of the wired connection and the wireless connection is available to connect the source control device 11 and the electronic cassette 13, 104. This does not intend just one of the wired connection and the wireless connection. The present invention includes the case of using both of the wired connection and the wireless connection together, as a matter of course. For example, a communication channel between the source control device 11 and the electronic cassette 104 (more particularly, the supplemental device 110) may be a mixture of the wireless method and the wired method.

Note that, the present invention is not limited to above embodiments, and is modified into various configurations within the scope of the present invention.

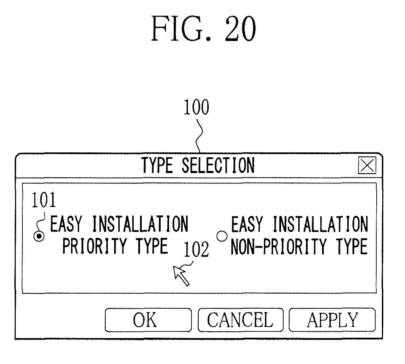

In the above embodiments, the source ID is transmitted and received upon establishing the communication between the source control device 11 and the console 14 after completely installing the X-ray imaging apparatus 2b, to retrieve and extract the region type of the X-ray source 10 corresponding to the source ID from the source information 99. However, the region type may be manually inputted by the operator. In this case, a type selection window 100, as shown in Fig. 20, is displayed on the display 89 of the console 14 or a monitor (not shown) of the electronic cassette 13. The type selection window 100 has radio buttons 101 for selecting one of the easy installation priority type (first AEC mode) and the easy installation non-priority type (second AEC mode). The operator chooses one of the two types by clicking on the radio button 101 using a pointer 102 or the like through the input device 90 or an operation section (not shown) of the electronic cassette 13. In a like manner, the source ID may be manually inputted by the operator, instead of being obtained automatically.

Also, the easy installation priority type and the easy installation non-priority type are set just as settings of the electronic cassette 13, and the maker of the electronic cassette 13 or a dealer thereof may set one of the types in advance in shipping. The electronic cassette 13 switches its operation in accordance with the set type. This eliminates time and effort to choose the type in a hospital being a customer. This also saves the maker from having to prepare software of both types for controlling the electronic cassette 13 and having to selectively install the software that adheres to each geographic region, and increases productivity.

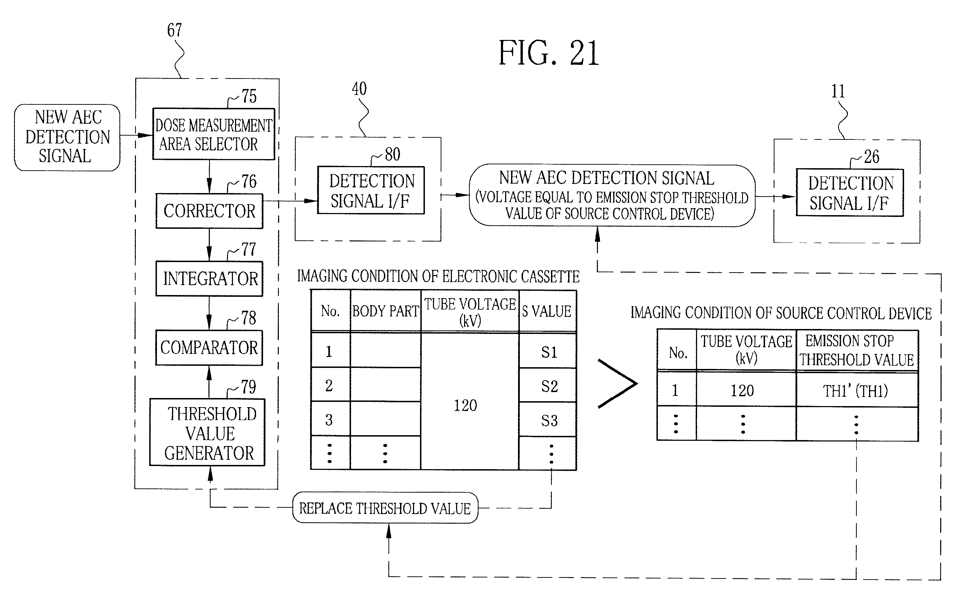

In the above embodiments, in a case where the region type is the easy installation priority type (first AEC mode), the detection signal I/F is set as the output destination, and the voltage value (new AEC detection signal) is set as the output format. The source control device 11, which has a limited number of imaging conditions (emission stop threshold values), makes a judgment on the stop of emission, so the image quality deteriorates more or less as compared with a case where the electronic cassette 13 performs the AEC in accordance with the precise imaging conditions. Accordingly, a contrivance as shown in Fig. 21 is made to perform the AEC based on the emission stop threshold values corresponding to the precise imaging conditions with the use of the detection signal I/F, in a case where source control device 11 has a less number of imaging conditions than those of the electronic cassette 13.

First, exactly the same process is carried out as the process of the first AEC mode in the first embodiment from the selection of the dose measurement area to the judgment of the stop of emission. However, the detection signal I/F 80 is used instead of the emission signal I/F 81. As soon as the integrated value of the detection signal has reached the emission stop threshold value produced by the threshold value generator 79, a voltage value that is equivalent to the emission stop threshold value (TH1', TH2, or the like of Fig. 2) of the source control device 11 at the corresponding tube voltage is transmitted through the detection signal I/F 80, instead of transmitting the emission stop signal through the emission signal I/F 81.

The emission stop threshold value produced by the threshold value generator 79 takes various values (see Fig. 6) even at the same tube voltage, in accordance with the imaging condition set in the console 14. Since the judgment of the stop of X-ray emission is made based on the threshold value corresponding to the imaging condition, the timing of the judgment varies depending on the imaging condition. According to this method, however, a signal to be transmitted from the electronic cassette 13 to the source control device 11 is only the voltage value (one type in this embodiment) equivalent to the emission stop threshold value of the source control device 11. In other words, the voltage value equivalent to the emission stop threshold value of the source control device 11 functions as the emission stop signal, the detection signal I/Fs 26 and 80 function as I/Fs dedicated to the transmission and reception of the emission stop signal. The electronic cassette 13 makes the judgment of the stop of emission in actual fact, but it is seen that the source control device 11 itself judges the stop of emission by receiving the voltage value equivalent to the emission stop threshold value.

This embodiment has both of an advantage of the easy installation priority type using the detection signal I/F 80 and an advantage of high image quality using the emission signal I/F 81 at the same time. This embodiment may be added as an easy installation and high image quality compatible type in the region type of the above embodiments. Note that, if the source control device 11 has two or more types of imaging conditions at the same tube voltage, the imaging conditions of the console 14 are grouped in advance, and each group is linked to one of the imaging conditions of the source control device 11 having the same tube voltage, so as to transmit a voltage value that is equivalent to the emission stop threshold value of the linked imaging condition of the source control device 11.

As described above, in the second AEC mode, the emission signal I/F 27 of the source control device 11 transmits and receives not only the emission stop signal (AEC signal) but also the signals other than the AEC signal, such as the emission start request signal and the emission permission signal, to and from the emission signal I/F 81 of the electronic cassette 13. Thus, a branch step for judging the type of a received signal and determining a course of a process is required and causes decrease in rapidity. Also there is a possibility of receiving the same type of signals in the same timing. This may cause a delay in the AEC, especially, in a process of the stop of X-ray emission. For example, in chest radiography, X-ray emission time is extremely short i.e. on the order of 50 ms, so the process of the stop of X-ray emission has to be performed rapidly.

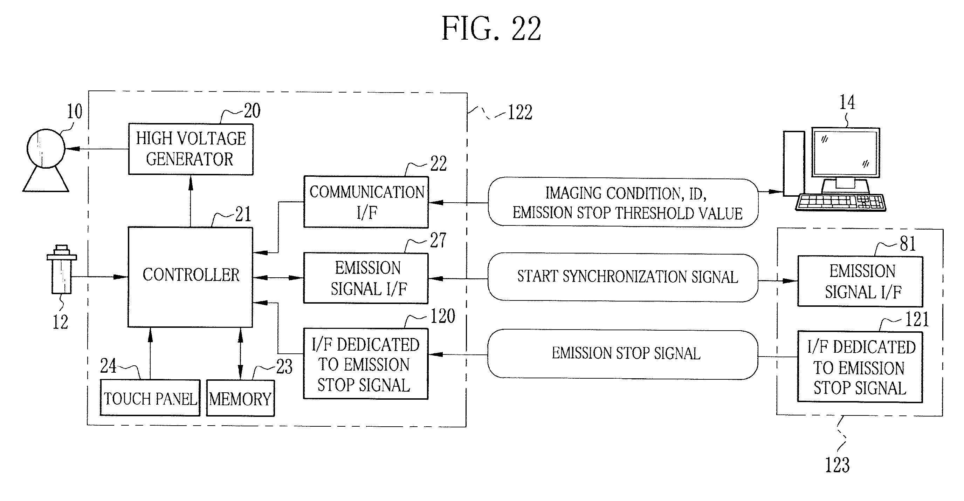

Accordingly, a source control device 122 and an electronic cassette 123 may be used, as shown in Fig. 22. The source control device 122 is provided with an I/F 120 dedicated to the emission stop signal, independently of the emission signal I/F 27, to transmit and receive only the emission stop signal therethrough. The electronic cassette 123 is provided with an I/F 121 dedicated to the emission stop signal to transmit and receive only the emission stop signal therethrough. In this case, the same process as that of the second AEC mode in the above embodiments is performed, but the I/Fs 120 and 121 dedicated to the emission stop signal, instead of the emission signal I/Fs 27 and 81, are necessarily in charge of the transmission and reception of the emission stop signal. Transmitting and receiving the emission stop signal, which is related to the judgment of the stop of X-ray emission, through the dedicated I/Fs independent of the I/Fs for transmitting the other signals eliminates the need for performing the branch process for judging the type of the signal and determining a process in accordance with the judgment. Also, it is possible to prevent the reception of the different types of signals in the same timing, so the process of the stop of X-ray emission can be made rapidly.

Note that, in transmitting and receiving the emission stop signal between the source control device and the electronic cassette, the source control device is prevented from receiving the different types of signals in the same timing by controlling that the electronic cassette does not transmit another signal. In this method, however, the signal transmission control of the electronic cassette becomes complicated. In this embodiment, since the emission stop signal related to the judgment of the stop of X-ray emission is transmitted and received through the dedicated I/Fs, the electronic cassette does not need to perform the signal transmission control and becomes simple.

Also, there are many cases that the X-ray generating apparatus and the X-ray imaging apparatus are made by different makers and the details of processes cannot be known each other. Thus, in the case of combining the X-ray source, the source control device, the electronic cassette, and the console made by the different makers, it is difficult to ensure that the X-ray emission stop process is performed without delay. In this embodiment, however, the emission stop signal related to the judgment of the stop of X-ray emission is transmitted and received through the dedicated I/Fs. Thus, if execution of the X-ray emission stop process without delay is ensured by assessing signal transmission performance of the electronic cassette and signal reception performance of the source control device, an operation of the system into which the above parts are combined is preferably ensured.

Although there is a problem of the complication more or less, as described above, with the aim of accelerating the X-ray emission stop process, not only the emission stop signal but also another signal may be transmitted through the I/Fs dedicated to the emission stop signal, only in a case where no collision between the signals is ensured in consideration of a sequence of the process of the system. This does not adversely affect the rapidity of the X-ray emission stop process in actual fact. More specifically, the start synchronization signal is never issued in timing of stopping the X-ray emission, and hence can be transmitted and received through the I/Fs dedicated to the emission stop signal. A signal that can be issued in arbitrary timing (irregular timing) such as a battery level check signal is transmitted and received through the different I/Fs.

Note that, in an example of Fig. 22, the signals other than the emission stop signal may be wirelessly transmitted and received between the emission signal I/F 27 of the source control device 11 and the emission signal I/F 81 of the electronic cassette 13, in addition to the wireless communication with the console 14. While the emission stop signal is securely transmitted and received through the wired communication, transmitting the other signals through the wireless communication secures the portability of the electronic cassette 13.

If a malfunction occurs in the detection pixel 65 of the electronic cassette 13 or the communication between the source control device 11 and the electronic cassette 13 is interrupted by a wiring disconnection, the AEC may not work due to a failure in the transmission and reception of the AEC signal. In the AEC, the source control device 11 sets as the X-ray emission time the maximum value allowable under safety restrictions, so a malfunction of the AEC poses the risk of excessive radiation exposure to a patient beyond the target dose. Therefore, the electronic cassette 13 has a test mode, and test radiography is performed in all the imaging conditions that the console 14 has, immediately after the installation and before radiography of one day. The detection pixels 65 keeps detecting the X-rays even after the electronic cassette 13 sends the AEC signal to the source control device 11. In a case where the stop of X-ray emission is detected within predetermined time, the AEC is judged to be performed normally. If not, it is judged that any breakdown occurs and a warning message is displayed on the display 89 of the console 14.

In a case where the source control device 11 and the electronic cassette 13 are connectable through both the wired and wireless communication between the detection signal I/Fs 26 and 80 or between the emission signal I/Fs 27 and 81, if the wireless communication is judged to be unstable as a result of monitoring radio field intensity or the like, a warning message may be displayed to recommend switching to the wired communication.

In the above embodiments, one X-ray generating apparatus 2a, one electronic cassette 13, and one console 14 are connected on a one-by-one basis for the sake of convenience in explanation. However, the present invention is intended for use in a case where one pair of X-ray generating apparatus and console is disposed in each examination room or each medically equipped vehicle and a several number of electronic cassettes are shared in the rooms or the vehicles, or one console performs centralized control of a plurality of X-ray generating apparatuses. In the former case, since an individual structure is the same as the structures of the above embodiments i.e. the connection on a one-by-one basis, the source ID is transmitted and received upon establishing the communication between the X-ray generating apparatus and the console, as with the above embodiments. In the latter case, the operator chooses which apparatus to use, out of the plurality of X-ray generating apparatuses, through the GUI (graphical user interface) on the display of the console, and the source ID of the chosen X-ray generating apparatus is transmitted and received between the X-ray generating apparatus and the console.

In the above embodiments, the source information 99 is stored in the storage device 87 of the console 14 and the region type, the correction information, and the like are transmitted from the console 14 to the electronic cassette 13, but the present invention is not limited to this. The source information 99 may be stored in an internal memory (not shown) of the controller 41 of the electronic cassette 13. In this case, the source ID is sent to the electronic cassette through the console. If there are a plurality of X-ray generating apparatuses, the electronic cassette may have information about the correlation between the source ID and a unique ID of the console or a wireless access point (in a case where the console and the electronic cassette are wirelessly connected) such as an IP address, an SSID, or an ESSID. The unique ID may be obtained upon connecting the console or the wireless access point, and the source ID corresponding to the obtained unique ID of the console or the wireless access point may be read out from the information on the correlation. In obtaining the unique ID of the wireless access point, a wireless access point that has the most favorable communication characteristics including the radio field intensity and the like is chosen. In the case of a medically quipped vehicle, a unique ID of the medically equipped vehicle may be used instead of the unique ID of the console or the wireless access point.

In the above embodiments, the detection pixel 65 that is directly connected to the signal line 52 without through the TFT 47 is used as the new AEC sensor. However, the radiation dose may be detected by monitoring an electric current flowing through the bias line 48 connected to a specific pixel 45, taking advantage of the fact that the electric current flowing through the bias line 48 for supplying the bias voltage Vb to each pixel 45 is based on electric charge produced in the pixel 45. Otherwise, the radiation dose may be detected based on a leak current that leaks from the pixel 45 when all the TFTs 47 are turned off. Furthermore, an AEC detection pixel that has a different structure and an independent output may be provided besides the pixels 45 in a plane coplanar to the imaging surface 36. Also, the radiation dose may be detected by nondestructive readout of the electric charge from the pixel by using a CMOS type image sensor as an FPD.

Note that, instead of integrating the detection signal by the integrator after the correction of the detection signal by the corrector, the integrated value of the detection signal outputted from the integrator may be corrected. In this case, the new AEC detection signal is inputted from the dose measurement area selector to the integrator, and the integrator calculates the integrated value. Then, the integrated value is inputted to the corrector to make a correction similar to the correction of the above embodiments.

In the above embodiments, the detection pixel 65 of the electronic cassette 13 is newly used as the AEC sensor, instead of the previous AEC sensor attached to the X-ray generating apparatus 2a, in other words, a retrofit. However, the present invention is applicable in the same manner to a case where the X-ray generating apparatus and the like are made by a maker while only the electronic cassette is made by an OEM supplier, because the OEM supplier of the electronic cassette has to change the output format of the AEC signal so as to be compatible with the format of the X-ray generating apparatus and the like made by the different maker.

The console 14 and the electronic cassette 13 are separate from each other in the above embodiments, but the console 14 is not necessarily an independent device, and the electronic cassette 13 has the function of the console 14. The present invention may be applied to an X-ray image detecting device to be mounted on the imaging stand, instead of or in addition to the electronic cassette, being a portable X-ray image detecting device.

In the above embodiments, the corrector 76 is provided to correct the new AEC detection signal to the detection signal corresponding to the previous AEC detection signal due to incompatibility in the specifications related to the AEC between the source control device and the electronic cassette. However, if the specifications are compatible, the corrector 76 is unnecessary.

The present invention is applicable to an imaging system using another type of radiation such as γ-rays, instead of the X-rays.

### DESCRIPTION OF THE REFERENCE NUMERALS

- 2: X-ray imaging system
- 10: X-ray source
- 11, 122: source control device
- 13, 105, 123, 151: electronic cassette
- 14: console
- 21: controller
- 25: previous AEC sensor
- 26, 80, 106, 116: detection signal I/F
- 27, 81, 117: emission signal I/F
- 35: FPD
- 40, 163: communication unit
- 41, 161: controller
- 45: pixel
- 65: detection pixel
- 67: AEC unit
- 75, 111: dose measurement area selector
- 76, 112: corrector
- 77, 113: integrator
- 78, 114: comparator
- 79, 115: threshold value generator
- 88: communication I/F
- 120, 121: I/F dedicated to emission stop signal
- 150: power feeding device
- 160: AEC controller
- 162: AEC communication unit

## Claims

1. A radiation imaging system **characterized in** comprising:
a radiation source for emitting radiation to an object;
a source control device for controlling an operation of said radiation source;
a radiographic image detecting device for detecting a radiographic image by receiving said radiation passed through said object, said radiographic image detecting device having an AEC sensor for performing automatic exposure control that detects a radiation dose passed through said object and stops a radiation emission from said radiation source as soon as an integrated value of said radiation dose has reached a predetermined emission stop threshold value;
a console for receiving said radiographic image detected by said radiographic image detecting device;
a high speed communication unit having a relatively high communication speed, for communicating an AEC signal related to said automatic exposure control between said source control device and said radiographic image detecting device; and
a low speed wireless communication unit having a communication speed lower than said communication speed of said high speed communication unit, for wirelessly communicating a signal other than said AEC signal between said radiographic image detecting device and said console.

2. The radiation imaging system according to claim 1, **characterized in that**
average delay time of data communication is small in said high speed communication unit; and
said delay time of said low speed wireless communication unit is larger than said delay time of said high speed communication unit.

3. The radiation imaging system according to claim 1 or 2, **characterized in that** said high speed communication unit performs wireless communication of said AEC signal.

4. The radiation imaging system according to claim 3, **characterized in that**
said high speed communication unit performs communication of said AEC signal by ad-hoc communications; and
said low speed wireless communication unit performs communication of said signal other than said AEC signal by infrastructure communications.

5. The radiation imaging system according to claim 4, **characterized in that** said radiographic image detecting device directly communicates said AEC signal with said source control device by said ad-hoc communications.

6. The radiation imaging system according to claim 1 or 2, **characterized in that** said high speed communication unit performs wired communication of said AEC signal.

7. The radiation imaging system according to claim 6, **characterized in that** said high speed communication unit also performs wired communication of said signal other than said AEC signal.

8. The radiation imaging system according to claim 7, **characterized in** comprising:
a judging section for judging whether or not to perform said automatic exposure control in radiography on an imaging condition in accordance with said imaging condition inputted through said console; and
a communication switching section for making said high speed communication unit, instead of said low speed wireless communication unit, communicate said signal other than said AEC signal, in a case where said judging section judges that said automatic exposure control is not performed.

9. The radiation imaging system according to one of claims 1 to 8, **characterized in that**
said high speed communication unit and said low speed wireless communication unit are made of different hardware resources; and
said radiographic image detecting device includes:
a first control section for performing control of a process and communication of said AEC signal; and
a second control section for performing control of a process and communication of said signal other than said AEC signal.

10. The radiation imaging system according to one of claims 1 to 9, **characterized in** comprising a low speed wired communication unit for performing wired communication of said signal other than said AEC signal at a communication speed lower than said communication speed of said high speed communication unit.

11. The radiation imaging system according to one of claims 1 to 10, **characterized in that**
said AEC signal is one of a dose detection signal of said AEC sensor and an emission stop signal that is outputted as soon as an integrated value of said dose detection signal of said AEC sensor has reached a predetermined emission stop threshold value; and
said radiographic image detecting device has two modes, including a first AEC mode for transmitting said dose detection signal of said AEC sensor to said source control device through said high speed communication unit and a second AEC mode for transmitting said emission stop signal.

12. The radiation imaging system according to claim 11, **characterized in that** said source control device and said radiographic image detecting device have, as said high speed communication unit, a detection signal I/F for communicating said dose detection signal and an emission signal I/F for communicating said emission stop signal.

13. The radiation imaging system according to claim 12, **characterized in that**
said radiographic image detecting device has a main body and a supplemental device, and said main body has an image detector for detecting said radiographic image and said AEC sensor, and said supplemental device has said detection signal I/F and said emission signal I/F; and
communication between said supplemental device and said main body adopts a same communication method as a communication method of said high speed communication unit.

14. The radiation imaging system according to one of claims 1 to 13, **characterized in that** said radiographic image detecting device is an electronic cassette having a portable housing.

15. The radiation imaging system according to claim 14, **characterized in that** said electronic cassette can be driven by a battery contained in said housing.

16. The radiation imaging system according to claim 15, **characterized in** comprising:
a noncontact power feeding device for supplying electric power to recharge said battery, wherein
said battery is rechargeable in a state of being contained in said electronic cassette with said electric power from said noncontact power feeding device.

17. The radiation imaging system according to claim 16, **characterized in that** said noncontact power feeding device is embedded in a holder of an imaging stand into which said electronic cassette is detachably loaded.

18. The radiation imaging system according to one of claims 1 to 17, **characterized in that**
said radiographic image detecting device includes an image detector that has an imaging surface and detects said radiographic image; and
said AEC sensor is disposed in said imaging surface.

19. A communication method of a radiation imaging system including:
a radiation source for emitting radiation to an object;
a source control device for controlling an operation of said radiation source;
a radiographic image detecting device for detecting a radiographic image by receiving said radiation passed through said object, said radiographic image detecting device having an AEC sensor for performing automatic exposure control that detects a radiation dose passed through said object and stops a radiation emission from said radiation source as soon as an integrated value of said radiation dose has reached a predetermined emission stop threshold value; and
a console for receiving said radiographic image detected by said radiographic image detecting device,
said communication method **characterized in** comprising:
a high speed communication step for communicating at a relatively high communication speed an AEC signal related to said automatic exposure control between said source control device and said radiographic image detecting device; and
a low speed wireless communication step for wirelessly communicating a signal other than said AEC signal between said radiographic image detecting device and said console at a communication speed lower than said communication speed of said AEC signal.

20. A radiographic image detecting device to be used in combination with a radiation source for emitting radiation to an object and a source control device for controlling an operation of said radiation source, for detecting a radiographic image by receiving said radiation passed through said object, said radiographic image detecting device **characterized in** comprising:
an AEC sensor for performing automatic exposure control that detects a radiation dose passed through said obj ect and stops a radiation emission from said radiation source as soon as an integrated value of said radiation dose has reached a predetermined emission stop threshold value;
a high speed communication unit for communicating an AEC signal related to said automatic exposure control with said source control device at a relatively high communication speed; and
a low speed wireless communication unit for wirelessly communicating a signal other than said AEC signal with a console for receiving said radiographic image at a communication speed lower than said communication speed of said AEC signal.

## Amended claims

### Amended claims under Art. 19.1 PCT

1. A radiation imaging system **characterized in** comprising:
a radiation source for emitting radiation to an object;
a source control device for controlling an operation of said radiation source;
a radiographic image detecting device for detecting a radiographic image by measuring said radiation passed through said object, said radiographic image detecting device having an AEC sensor for performing automatic exposure control that stops a radiation emission from said radiation source based on a radiation dose passed through said object;
a console for receiving said radiographic image from said radiographic image detecting device;
a high speed communication unit having a relatively high communication speed, for communicating an AEC signal related to said automatic exposure control between said source control device and said radiographic image detecting device; and
a low speed wireless communication unit having a communication speed lower than said communication speed of said high speed communication unit, for wirelessly communicating a signal other than said AEC signal between said radiographic image detecting device and said console.

2. The radiation imaging system according to claim 1, **characterized in that**
average delay time of data communication is small in said high speed communication unit; and
said delay time of said low speed wireless communication unit is larger than said delay time of said high speed communication unit.

3. The radiation imaging system according to claim 1 or 2,
1 **characterized in that** said high speed communication unit performs wireless communication of said AEC signal.

4. The radiation imaging system according to claim 3, **characterized in that**
said high speed communication unit performs communication of said AEC signal by ad-hoc communications; and
said low speed wireless communication unit performs communication of said signal other than said AEC signal by infrastructure communications.

5. The radiation imaging system according to claim 4, **characterized in that** said radiographic image detecting device directly communicates said AEC signal with said source control device by said ad-hoc communications.

6. The radiation imaging system according to claim 1 or 2, **characterized in that** said high speed communication unit performs wired communication of said AEC signal.

7. The radiation imaging system according to claim 6, **characterized in that** said high speed communication unit also performs wired communication of said signal other than said AEC signal.

8. (currently amended) The radiation imaging system according to claim 7 **characterized in** further comprising:
a judging section for judging whether or not to perform said automatic exposure control in radiography in accordance with an imaging condition inputted through said console; and
a communication switching section for making said high speed communication unit, instead of said low speed wireless communication unit, communicate said signal other than said AEC signal, in a case where said judging section judges that said automatic exposure control is not performed.

9. The radiation imaging system according to one of claims 1 to 8, **characterized in that**
said high speed communication unit and said low speed wireless communication unit are made of different hardware resources; and
said radiographic image detecting device includes a first control section for performing control of a process and communication of said AEC signal and a second control section for performing control of a process and communication of said signal other than said AEC signal.

10. The radiation imaging system according to one of claims 1 to 9, **characterized in** further comprising:
a low speed wired communication unit for performing wired communication of said signal other than said AEC signal at a communication speed lower than said communication speed of said high speed communication unit.

11. The radiation imaging system according to one of claims 1 to 10, **characterized in that**
said AEC signal is one of a dose detection signal of said AEC sensor and an emission stop signal that is outputted as soon as an integrated value of said dose detection signal of said AEC sensor has reached a predetermined emission stop threshold value; and
said radiographic image detecting device has two modes, including a first AEC mode for transmitting said dose detection signal of said AEC sensor and a second AEC mode for transmitting said emission stop signal to said source control device through said high speed communication unit.

12. The radiation imaging system according to claim 11, **characterized in that** as said high speed communication unit, each of said source control device and said radiographic image detecting device has a detection signal I/F for communicating said dose detection signal and an emission signal I/F for communicating said emission stop signal.

13. The radiation imaging system according to claim 12, **characterized in that**
said radiographic image detecting device has a main body and a supplemental device, and said main body has an image detector for detecting said radiographic image and said AEC sensor, and said supplemental device has said detection signal I/F and said emission signal I/F; and
communication between said supplemental device and said main body adopts a same communication method as a communication method of said high speed communication unit.

14. The radiation imaging system according to one of claims 1 to 13, **characterized in that** said radiographic image detecting device is an electronic cassette having a portable housing.

15. The radiation imaging system according to claim 14, **characterized in that** said electronic cassette can be driven by a battery contained in said housing.

16. The radiation imaging system according to claim 15 **characterized in** further comprising:
a noncontact power feeding device for supplying electric power to recharge said battery, wherein
said battery is rechargeable in a state of being contained in said electronic cassette with said electric power from said noncontact power feeding device.

17. The radiation imaging system according to claim 16, **characterized in that** said noncontact power feeding device is embedded in a holder of an imaging stand into which said electronic cassette is detachably loaded.

18. The radiation imaging system according to one of claims 1 to 17, **characterized in that**
said radiographic image detecting device includes an image detector that has an imaging surface and detects said radiographic image; and
said AEC sensor is disposed in said imaging surface.

19. (currently amended) A communication method of a radiation imaging system including:
a radiation source for emitting radiation to an object;
a source control device for controlling an operation of said radiation source;
a radiographic image detecting device for detecting a radiographic image by measuring said radiation passed through said object, said radiographic image detecting device having an AEC sensor for performing automatic exposure control that stops a radiation emission from said radiation source based on a radiation dose passed through said object; and
a console for receiving said radiographic image from said radiographic image detecting device,
said communication method **characterized in** comprising:
a high speed communication step for communicating at a relatively high communication speed an AEC signal related to said automatic exposure control between said source control device and said radiographic image detecting device; and
a low speed communication step for wirelessly communicating a signal other than said AEC signal between said radiographic image detecting device and said console at a communication speed lower than said communication speed of said AEC signal.

20. (currently amended) A radiographic image detecting device to be used in combination with a radiation source for emitting radiation to an object and a source control device for controlling an operation of said radiation source, for detecting a radiographic image by measuring said radiation passed through said object, said radiographic image detecting device **characterized in** comprising:
an AEC sensor for performing automatic exposure control that stops a radiation emission from said radiation source based on a radiation dose passed through said object;
a high speed communication unit for communicating an AEC signal related to said automatic exposure control with said source control device at a relatively high communication speed; and
a low speed wireless communication unit for wirelessly communicating a signal other than said AEC signal with a console for receiving said radiographic image at a communication speed lower than said communication speed of said AEC signal.

21. (new) The radiation imaging system according to one of claims 1 to 18, **characterized in that** in said automatic exposure control, said radiation emission is stopped as soon as an integrated value of said radiation dose detected by said AEC sensor has reached a predetermined emission stop threshold value.

22. (new) A radiation imaging system **characterized in** comprising:
a radiation source for emitting radiation to an object;
a source control device for controlling an operation of said radiation source;
a radiographic image detecting device for detecting a radiographic image by measuring said radiation passed through said object, said radiographic image detecting device having an AEC sensor for performing automatic exposure control that stops a radiation emission from said radiation source based on a radiation dose passed through said object;
a console for receiving said radiographic image from said radiographic image detecting device;
a high speed communication unit having small average delay time of data communication, for communicating an AEC signal related to said automatic exposure control between said source control device and said radiographic image detecting device; and
a low speed wireless communication unit having delay time larger than said delay time of said high speed communication unit, for wirelessly communicating a signal other than said AEC signal between said radiographic image detecting device and said console.

23. (new) The radiation imaging system according to claim 22, **characterized in that** said high speed communication unit wirelessly communicates said AEC signal.

24. (new) The radiation imaging system according to claim 23, **characterized in that**
said high speed communication unit performs communication of said AEC signal by ad-hoc communications; and
said low speed wireless communication unit performs communication of said signal other than said AEC signal by infrastructure communications.

25. (new) The radiation imaging system according to claim 24, **characterized in that** said radiographic image detecting device directly communicates said AEC signal with said source control device by said ad-hoc communications.

26. (new) The radiation imaging system according to claim 22, **characterized in that** said high speed communication unit performs wired communication of said AEC signal.

27. (new) The radiation imaging system according to claim 26, **characterized in that** said high speed communication unit also performs wired communication of said signal other than said AEC signal.

28. (new) The radiation imaging system according to claim 27 **characterized in** further comprising:
a judging section for judging whether or not to perform said automatic exposure control in radiography in accordance with an imaging condition inputted through said console; and
a communication switching section for making said high speed communication unit, instead of said low speed wireless communication unit, communicate said signal other than said AEC signal, in a case where said judging section judges that said automatic exposure control is not performed.

29. (new) The radiation imaging system according to one of claims 22 to 28, **characterized in that**
said high speed communication unit and said low speed wireless communication unit are made of different hardware resources; and
said radiographic image detecting device includes a first control section for performing control of a process and communication of said AEC signal and a second control section for performing control of a process and communication of said signal other than said AEC signal.

30. (new) The radiation imaging system according to one of claims 22 to 29, **characterized in** further comprising:
a low speed wired communication unit for performing wired communication of said signal other than said AEC signal at a communication speed lower than said communication speed of said high speed communication unit.

31. (new) The radiation imaging system according to one of claims 22 to 30, **characterized in that**
said AEC signal is one of a dose detection signal of said AEC sensor and an emission stop signal that is outputted as soon as an integrated value of said dose detection signal of said AEC sensor has reached a predetermined emission stop threshold value; and
said radiographic image detecting device has two modes, including a first AEC mode for transmitting said dose detection signal of said AEC sensor and a second AEC mode for transmitting said emission stop signal to said source control device through said high speed communication unit.

32. (new) The radiation imaging system according to claim 31, **characterized in that** as said high speed communication unit, each of said source control device and said radiographic image detecting device has a detection signal I/F for communicating said dose detection signal and an emission signal I/F for communicating said emission stop signal.

33. (new) The radiation imaging system according to claim 32, **characterized in that**
said radiographic image detecting device has a main body and a supplemental device, and said main body has an image detector for detecting said radiographic image and said AEC sensor, and said supplemental device has said detection signal I/F and said emission signal I/F; and
communication between said supplemental device and said main body adopts a same communication method as a communication method of said high speed communication unit.

34. (new) The radiation imaging system according to one of claims 22 to 33, **characterized in that** said radiographic image detecting device is an electronic cassette having a portable housing.

35. (new) The radiation imaging system according to claim 34, **characterized in that** said electronic cassette can be driven by a battery contained in said housing.

36. (new) The radiation imaging system according to one of claims 22 to 35, **characterized in that** in said automatic exposure control, said radiation emission is stopped as soon as an integrated value of said radiation dose detected by said AEC sensor has reached a predetermined emission stop threshold value.

37. (new) A communication method of a radiation imaging system including:
a radiation source for emitting radiation to an object;
a source control device for controlling an operation of said radiation source;
a radiographic image detecting device for detecting a radiographic image by measuring said radiation passed through said object, said radiographic image detecting device having an AEC sensor for performing automatic exposure control that stops a radiation emission from said radiation source based on a radiation dose passed through said object; and
a console for receiving said radiographic image from said radiographic image detecting device,
said communication method **characterized in** comprising:
a high speed communication step for communicating an AEC signal related to said automatic exposure control between said source control device and said radiographic image detecting device with small average delay time of data communication; and
a low speed wireless communication step for wirelessly communicating a signal other than said AEC signal between said radiographic image detecting device and said console with delay time larger than said delay time of the communication of said AEC signal.

38. (new) A radiographic image detecting device to be used in combination with a radiation source for emitting radiation to an object and a source control device for controlling an operation of said radiation source, for detecting a radiographic image by measuring said radiation passed through said object, said radiographic image detecting device **characterized in** comprising:
an AEC sensor for performing automatic exposure control that stops a radiation emission from said radiation source based on a radiation dose passed through said object;
a high speed communication unit having small average delay time of data communication, for communicating an AEC signal related to said automatic exposure control with said source control device; and
a low speed wireless communication unit having delay time larger than said delay time of said high speed communication unit, for wirelessly communicating a signal other than said AEC signal with a console for receiving said radiographic image.

Statement under Art. 19.1 PCT
As to claims 1, 19, and 20, the expression of "as soon as an integrated value of said radiation dose has reached a predetermined emission stop threshold value" was deleted. The deleted part was converted into claim 21. Also, the expression of "on an imaging condition" of claim 8 was deleted. The deletion does not introduce any new technical matter, nor deletes constituent features required for solving an object of the present invention.

In claims 1, 19, and 20, "by receiving said radiation passed through said object" was changed to "by measuring said radiation passed through said obj ect" , and "detects a radiation dose passed through said object" was changed to "based on a radiation dose passed through said object". Also, in claims 1 and 19, "said radiographic image detected by said radiographic image detecting device" was changed to "said radiographic image from said radiographic image detecting device".

In claim 22, the expressions of "a high speed communication unit having a relatively high communication speed" and "a low speed wireless communication unit having a communication speed lower than said communication speed of said high speed communication unit" of claim 1 were replaced with "a high speed communication unit having small average delay time of data communication" and "a low speed wireless communication unit having delay time larger than said delay time of said high speed communication unit", respectively.

The contents of claims 23 to 35 are the same as those of claims 3 to 15.

The contents of claim 36 are the same as that of claim 21.

In claim 37, "a radiation imaging system" of claim 22 is replaced with "a communication method of a radiation imaging system".

In claim 38, "a radiation imaging system" of claim 22 is replaced with "a radiographic image detecting device".
